# NEW EUROPEAN PATENT SPECIFICATION

(11) **EP 4 153 133 B2**
(45) Date of publication and mention of the opposition decision: **06.05.2026**
(45) Mention of the grant of the patent: 08.11.2023
(21) Application number: 21727821.7
(22) Date of filing: 19.05.2021
(51) Int. Cl.: A61K 9/00, A61K 9/10, A61K 9/14, A61K 9/16, A61K 9/50, A61K 31/422, A61K 31/7028, A61P 33/00, A61P 33/10, A61P 33/14

(54) **INJECTABLE PHARMACEUTICAL COMPOSITIONS AND USES THEREOF**
INJIZIERBARE PHARMAZEUTISCHE ZUSAMMENSETZUNGEN UND VERWENDUNGEN DAVON
COMPOSITIONS PHARMACEUTIQUES INJECTABLES ET LEURS UTILISATIONS

(30) Priority: 20.05.2020 EP 20175597
(43) Date of publication of application: 29.03.2023
(73) Proprietor: Intervet International B.V., 5831 AN Boxmeer (NL)
(72) Inventor: VALLE COLON, Brenda, L, Rahway, NJ 07065 (US); FREEHAUF, Keith, Madison, NJ 07940 (US); KULCZAR, Christopher, D, Rahway, NJ 07065 (US); GUERINO, Frank, Madison, NJ 07940 (US)
(74) Representative: Intervet International B.V.
(86) International application number: PCT/EP2021/063236
(87) International publication number: WO 2021/233967

(56) References cited:
- WO-A1-2009/053466
- WO-A1-2015/048371
- WO-A1-2016/138339
- WO-A1-2019/050259
- KR-A- 20190 027 349
- NADJA ROHDICH ET AL: "Field effectiveness and safety of fluralaner plus moxidectin (Bravecto Plus) against ticks and fleas: a European randomized, blinded, multicenter field study in naturally-infested client-owned cats", PARASITES & VECTORS, vol. 11, no. 1, 19 November 2018 (2018-11-19), XP055714293, DOI: 10.1186/s13071-018-3175-z

## Description

The present invention relates to injectable veterinary pharmaceutical compositions comprising the combination of an isoxazoline compound and a physiologically active macrocyclic lactone.

### Background of the Invention

A number of parasites can infest or infect domestic animals, especially companion animals such as cats and dogs. These parasites are of great nuisance to both the animals and their owners.

Isoxazoline compounds are known in the art and the preparation of these compounds and their use as an antiparasitic are described, for example, in US patent application US 2007/0066617 and international patent applications WO 2005/085216, WO 2007/079162, WO 2009/002809, WO 2009/024541, WO 2009/003075, WO 2009/080250, WO 2010/070068, WO 2010/079077 and WO 2011/124998.

Injectable formulations of isoxazoline compounds have been described. WO 2015/048371 discloses long acting injectable compositions comprising spirocyclic isoxazoline compounds, one biopolymer and at least one carrier, solvent or excipient. WO 2016/138339 discloses long acting injectable formulations for comprising at least one isoxazoline active agent, a poloxamer and a co-solvent. WO 2016/164487 discloses extended release injectable veterinary formulations comprising at least one isoxazoline active agent, a pharmaceutically acceptable polymer and a solvent for use against parasites.

U.S. patent No. 9,609,869 discloses insecticidal compounds based on isoxazoline derivatives for use in controlling pest associated with agriculture, horticulture, animal husbandry and companion animals.

US patent application publication No. 2017/0239218 discloses long acting injectable compositions for combating parasites comprising at least one isoxazoline active agent, a liquid PEG and/or a neutral oil.

Further, physiologically active macrocyclic lactones are known for controlling parasite infestations. For example, moxidectin is useful for the prevention and treatment of infections and infestations caused by helminths, nematodes, and ectoparasitic arthropods.

Moxidectin was disclosed in U.S. patent number 4,916,154 and EP 0 525 307 and EP 1 197 207 disclose moxidectin microspheres and injectable compositions and their preparation and their use.

### Summary of the invention

In one aspect the subject of the present invention is directed to an injectable veterinary pharmaceutical composition comprising
(a) microspheres comprising
   (a1) about 1% to about 40% w/w based on the weight of the microspheres, of one or more physiologically active macrocyclic lactone(s), and
   (a2) about 60% to about 99% w/w, based on the weight of the microspheres, of polycaprolactone (PCL), and
(b) particles of an isoxazoline compound of Formula (I) 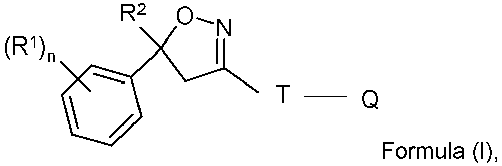 wherein
   R¹ = halogen, CF₃, OCF₃, CN,
   n = integer from 0 to 3, preferably 1, 2 or 3,
   R² = C₁-C₃-haloalkyl, preferably CF₃ or CF₂Cl,
   T = 5 to 12-membered mono or bicyclic ring system which is optionally substituted by one or more radicals Y,
   Y = methyl, halomethyl, halogen, CN, NO₂, NH₂-C=S, or two adjacent radicals Y form together a chain, especially a three or four-membered chain;
   Q = X-NR³R⁴ ora 5-membered N-heteroaryl ring which is optionally substituted by one or more radicals;
   X = CH₂, CH(CH₃), CH(CN), CO, CS,
   R³ = hydrogen, methyl, haloethyl, halopropyl, halobutyl, methoxymethyl, methoxyethyl, halomethoxymethyl, ethoxymethyl, haloethoxymethyl, propoxymethyl, ethylaminocarbonylmethyl, ethylaminocarbonylethyl, dimethoxyethyl, propynylaminocarbonylmethyl, N-phenyl-N-methyl-amino, haloethylaminocarbonylmethyl, haloethylaminocarbonylethyl, tetrahydrofuryl, methylaminocarbonylmethyl, (N,N-dimethylamino)-carbonylmethyl, propylaminocarbonylmethyl, cyclopropylaminocarbonylmethyl, propenylaminocarbonylmethyl, haloethylaminocarbonylcyclopropyl, 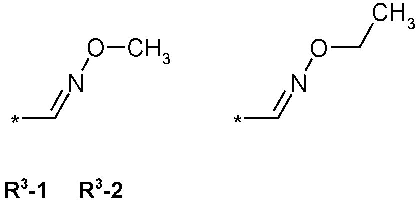 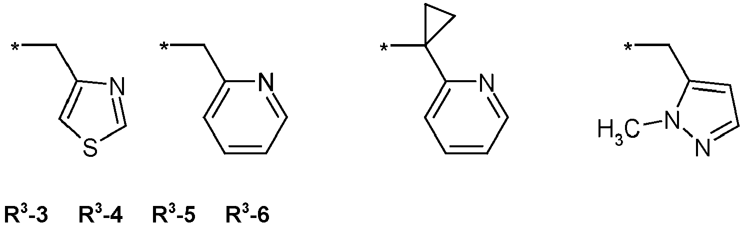 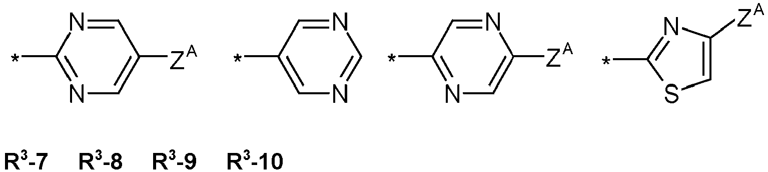 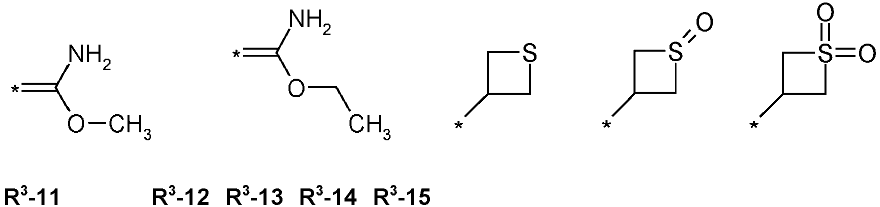 wherein Z^{A} = hydrogen, halogen, cyano, halomethyl (CF₃);
   R⁴ = hydrogen, ethyl, methoxymethyl, halomethoxymethyl, ethoxymethyl, haloethoxymethyl, propoxymethyl, methylcarbonyl, ethylcarbonyl, propylcarbonyl, cyclopropylcarbonyl, methoxycarbonyl, methoxymethylcarbonyl, aminocarbonyl, ethylaminocarbonylmethyl, ethylaminocarbonylethyl, dimethoxyethyl, propynylaminocarbonylmethyl, haloethylaminocarbonylmethyl, cyanomethylaminocarbonylmethyl, or haloethylaminocarbonylethyl; or
   R³ and R⁴ together form a substituent selected from the group consisting of: 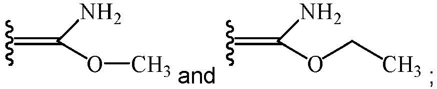 and
c) an aqueous carrier;
wherein the microspheres (a) and the particles of compound (b) are suspended in the aqueous carrier (c).

In another aspect the subject of the present invention is directed to a method of preparing such injectable veterinary composition comprising the steps of:
i) preparing microspheres (a) of a macrocyclic lactone by solvent evaporation, spinning disk atomization or spray drying, and optionally sieving the resulting product,
ii) preparing particles (b) of the isoxazoline compound of Formula (I),
iii) preparing the aqueous carrier by dissolving one or more suspending agents and/or one or more wetting agents in water, and
iv) mixing the particles obtained from step i) and the microspheres obtained from step ii) with the aqueous carrier obtained in step iii).

In another aspect the subject of the present invention is directed to a kit, wherein the kit comprises:
(A) a first container comprising a mixture of microspheres and particles of an isoxazoline compound of Formula (I) as defined above,
(B) a second container with an aqueous carrier as defined above; and
(C) instructions for reconstituting the microspheres and the particles in the aqueous carrier prior to an injection to an animal.

In another aspect the subject of the present invention is directed to the injectable veterinary composition as defined above for use in treating and/or preventing a parasite infestation in an animal.

### Description of the Figures

Figure 1: Scanning Electron Micrograph of microspheres comprising moxidectin and polycaprolactone (PCL) prepared by solvent evaporation.
Figure 2: Scanning Electron Micrograph of microspheres comprising moxidectin and polycaprolactone (PCL) prepared by spinning disk atomization (using dichloromethane).
Figure 3: Scanning Electron Micrograph of microspheres comprising moxidectin and polycaprolactone (PCL) prepared by spinning disk atomization (using acetone).
Figure 4: Scanning Electron Micrograph of microspheres comprising moxidectin and polycaprolactone (PCL) prepared by spray drying.
Figure 5: Scanning Electron Micrograph of microspheres comprising 50% Fluralaner and polylactic-co-glycolic acid (PLGA)
Figure 6: Mean fluralaner plasma concentration in dogs following administration of the present injectable veterinary composition.
Figure 7: Mean moxidectin plasma concentration in dogs following administration of the present injectable veterinary composition.
Figure 8: Mean fluralaner plasma concentration in dogs following administration of different fluralaner and moxidectin suspensions.

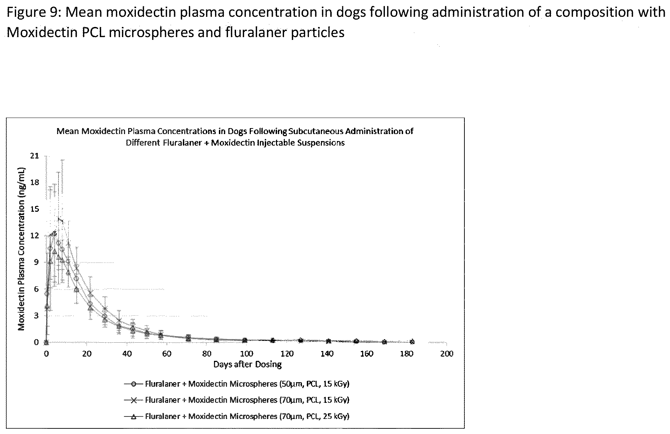

Figure 9: Mean moxidectin plasma concentration in dogs following administration of different fluralaner and moxidectin suspensions.

### Detailed description

It is an unmet veterinary medical need to protect animals, especially companion animals for a long time with a single injection against the most important parasites.

This means a long-term protection against ectoparasites such as fleas, ticks and mites and helminth parasites, such as heartworm and certain gastrointestinal heminths. In order to keep or alternatively enhance/improve the prophylactic and/or therapeutic effect of an injectable veterinary pharmaceutical composition, it would be desirable to have a long acting dosage form, comprising one or more active agents from a different antiparasitic class.

In particular, an advantageous injectable pharmaceutical composition for veterinary application is desirable, that enables a single injection to provide efficacious concentration levels of both classes of active compounds, an isoxazoline compound as well as a macrocyclic lactone such as moxidectin, in blood plasma of the treated animals over an extended period. Specifically, a feasible injectable composition that allows the effective and safe release of an effective amount of an isoxazoline compound as described above as well as a macrocyclic compound such as moxidectin in a combined composition safely and effectively over a prolonged time would be desirable.

For high efficacy and bioavailability of pharmaceuticals, it may be necessary to reach high serum levels of active substances in a very short time, which requires dosage forms that are released as quickly as possible. However, rapid release is often hampered by the poor water-solubility of active substances.

Besides the duration of action there are further desirable features for such injectable veterinary compositions such as: easiness of application (syringeability and re-suspendability), the possibility to sterilize the composition and/or the absence of side effects such as local injection site reaction and systemic side effects following administration.

Another object is to provide a composition ensuring a stable content of the active ingredients in such dosage forms, especially the macrocyclic compound such as moxidectin.

The inventors of the present invention identified new injectable compositions in the form of an aqueous suspension as described herein with favorable properties that address one or more of such desires.

We have now found injectable compositions of a combination of an isoxazoline compound of Formula (I) or a compound of formula (II) and one or more of a macrocyclic lactone which have long duration of activity, and provide long plasma levels in vivo and which have beneficial properties , e.g. injection site tolerability, stability on storage, acceptable viscosity, syringeability across a wide temperature range and good bioavailability.

Aqueous suspension means a composition that comprises particles that are mixed with, but undissolved in an aqueous carrier (also referred to as liquid aqueous vehicle or inert medium) comprising water used as a solvent (or diluent) in which the active agent is formulated and/ or administered. The aqueous carrier may contain some excipients, for example one or more suspending agents and/or one or more wetting agents.

A particle is a mobile, undissolved, solid matter that is suspended in a liquid. In this application the term encompasses both crystalline (or alternatively) amorphous solid forms of isoxazoline compounds as particles and microspheres as particles.

In one embodiment the particles of the isoxazoline compound or the compound of Formula (II) are crystalline.

Preferably the particles of the isoxazoline compound are manufactured according to the method as described in WO 2019/091940 A1 or as described in the examples.

In another embodiment the isoxazoline compound particles are present as microspheres, especially PLGA

Microsphere are small spherical particle having the particle size range 0.1-200µm and made up of biodegradable and non-biodegradable material and active ingredients and can be injected.

It has been found that it is advantageous to include the macrocyclic lactone as microspheres prepared with polycaprolactone.

One important aspect of the present invention is an injectable composition comprising (a) microspheres comprising (a1) about 1% to about 40% by weight of one or more physiologically active macrocyclic lactone(s), and (a2) about 60% to about 99% by weight polycaprolactone (PCL), based on the weight of the microspheres with (b) particles of an isoxazoline compound of Formula (I) and/or a compound according to Formula (II), wherein the microspheres (a) and compound (b) are suspended in an aqueous carrier (c) comprising one or more wetting agents and/or one or more suspending agents and water.

The injectable veterinary composition comprises component (a), microspheres, comprising (a1) about 1% to about 40% by weight of one or more physiologically active macrocyclic lactone(s) and (a2) about 60% to about 99% by weight of polycaprolactone (PCL), both based on the weight of the microspheres.

In a preferred embodiment of the invention and/or embodiments thereof the microspheres (a) comprise the about 1% by weight to about 40% by weight, preferably about 2 % by weight to about 35% by weight, more preferably about 3 % by weight to about 30% by weight, in particular about 5% by weight to about 20% by weight, especially about 8 % to 12% , especially 10% by weight of one or more physiologically active macrocyclic lactone(s) (a1) based on the weight of the microspheres (a).

In a preferred embodiment of the invention and/or embodiments thereof, the present composition comprises one or more physiologically active macrocyclic lactone(s) in an amount of about 0.01% by weight to about 1.0% by weight, more preferably of about 0.05% by weight to about 0.85% by weight, even more preferably of about 0.1% by weight to about 0.7% by weight, in particular preferably of about 0.15% by weight to about 0.5% by weight, especially about 0.17% by weight, based on the total weight of the composition.

In an embodiment of the invention and/or embodiments thereof the one or more physiologically active macrocyclic lactone(s) (a1) include(s), but is not limited to, avermectins or milbemycins. Such avermectins or milbemycins are known.

In an embodiment of the invention and/or embodiments thereof, the physiologically active macrocyclic lactone(s) (a1) is selected from ivermectin, abamectin, milbemycin oxime, moxidectin, milbemectine, nemadectin, milbemycin-D, doramectin, selamectin, eprinomectin, emamectin and mixtures thereof.

In a preferred embodiment of the invention and/or embodiments thereof, the physiologically active macrocyclic lactone(s) (a1) is moxidectin.

In a preferred embodiment of the invention and/or embodiments thereof, the microspheres (a) comprise the about 60% by weight to about 99% by weight, preferably about 65% by weight to about 98% by weight, more preferably about 70 % by weight to about 97% by weight, in particular about 80% by weight to about 95% by weight, especially about 90% weight of (a2) polycaprolactone (PCL), based on the weight of the microspheres (a).

In a preferred embodiment of the invention and/or embodiments thereof the microspheres (a) comprise about 60% by weight, about 65% by weight, about 70% by weight, about 72% by weight, about 75% by weight, about 78% by weight, about 80% by weight, about 81% by weight, about 82% by weight, about 83% by weight, about 84% by weight, about 85% by weight, about 86% by weight, about 87% by weight, about 88% by weight, about 90% by weight, about 91 % by weight, about 92% by weight, about 93% by weight, about 94% by weight, about 95% by weight, about 96% by weight, about 97% by weight, about 98% by weight or about 99% of (a2) polycaprolactone (PCL), by weight based on the weight of the microspheres (a).

Without being bound to any theory, it appears the volume weighted particle size of the microspheres (a) and the particle size of the isoxazoline compound is of importance with regard to the administration of the present composition. The particle size of any ingredient, in particular the active ingredient in the suspension, can influence the re-suspendability and syringeability.

For this purpose, the particle size should be small enough to prevent compaction or caking and to facilitate re-suspension and reconstitution without causing any negative effect when administered to an animal.

As used herein, particle size data reported herein refers to volume weighted particle size as measured by conventional particle techniques well known to those skilled in the art, such as static light scattering (also known as laser diffraction), image analysis or sieving. More discussion of particle size measurement is provided below.

The volume weighted particle size can be measured by sieving, microscopy or laser diffraction (Malvern or Sympatec). The volume weighted particle size measurement can be performed with a Malvern Mastersizer 2000 with the Hydro 2000G measuring cell, or with a Horiba LA-910 laser scattering particle size distribution analyzer. The volume weighted particle size can be measured by a Sympatec Helos instrument. Preferably a Malvern Mastersizer 2000 with Scirocco 2000 dry sizing feed attachment for particle size analysis is used.

Particle size distribution describes the relative amounts of particles present according to the size.

The average particle size (D10), which is also denoted D10 value of the integral volume distribution, is defined in the context of this invention as the particle diameter at which 10 percent by volume of the particles have a smaller diameter than the diameter which corresponds to the D10 value. Likewise, 90 percent by volume of the particles have a larger diameter than the D10 value.

The average particle size (D50), which is also denoted D50 value of the integral volume distribution, is defined in the context of this invention as the particle diameter at which 50 percent by volume of the particles have a smaller diameter than the diameter which corresponds to the D50 value. Likewise, 50 percent by volume of the particles have a larger diameter than the D50 value.

The average particle size (D90), which is also denoted D90 value of the integral volume distribution, is defined in the context of this invention as the particle diameter at which 90 percent by volume of the particles have a smaller diameter than the diameter which corresponds to the D90 value. Likewise, 10 percent by volume of the particles have a larger diameter than the D90 value.

In an embodiment of the invention or embodiments thereof, the D10 of the volume weighted particle size distribution of the microspheres (a) is from about 2 µm to about 130 µm, more preferably from about 3 µm to about 115 µm, even more preferably from about 5 µm to about 100 µm, in particular from about 10 µm to about 50 µm.

In an embodiment of the invention or embodiments thereof, the D10 of the volume weighted particle size distribution of the microspheres (a) is from about 2 µm to about 10 µm, more preferably from about 3 µm to about 8 µm, in particular from about 4 µm to about 6 µm.

In an embodiment of the invention or embodiments thereof, the D50 of the volume weighted particle size distribution of the microspheres (a) is from about 8 µm to about 250 µm, more preferably from about 20 µm to about 200 µm, even more preferably from about 40 µm to about 80 µm, in particular from about 50 µm to about 70 µm.

In an embodiment of the invention or embodiments thereof, the D90 of the volume weighted particle size distribution of the microspheres (a) is from about 25 µm to about 325 µm, more preferably from about 35 µm to about 300 µm, even more preferably from about 50 µm to about 280 µm, in particular from about 100 µm to about 150 µm.

The injectable veterinary composition comprises component (b), namely particles of an isoxazoline compound of Formula (I) 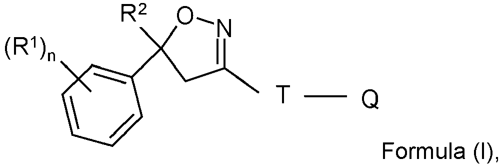 wherein
- R¹ =: halogen, CF₃, OCF₃, CN,
- n =: integer from 0 to 3, preferably 1, 2 or 3,
- R² =: C₁-C₃-haloalkyl, preferably CF₃ or CF₂Cl,
- T =: 5 to 12-membered mono or bicyclic ring system which is optionally substituted by one or more radicals Y,
- Y =: methyl, halomethyl, halogen, CN, NO₂, NH₂-C=S, or two adjacent radicals Y form together a chain, especially a three or four-membered chain,
- Q =: X-NR³R⁴ ora 5-membered N-heteroaryl ring which is optionally substituted by one or more radicals,
- X =: CH₂, CH(CH₃), CH(CN), CO, CS,
- R³ =: hydrogen, methyl, haloethyl, halopropyl, halobutyl, methoxymethyl, methoxyethyl, halomethoxymethyl, ethoxymethyl, haloethoxymethyl, propoxymethyl, ethylamino-carbonylmethyl, ethylaminocarbonylethyl, dimethoxyethyl, propynylamino-carbonylmethyl, N-phenyl-N-methyl-amino, haloethylaminocarbonylmethyl, halo-ethylaminocarbonylethyl, tetrahydrofuryl, methylaminocarbonylmethyl, (N,N-di-methylamino)-carbonylmethyl, propylaminocarbonylmethyl, cyclopropylamino-carbonylmethyl, propenylaminocarbonylmethyl, haloethylaminocarbonylcyclo-propyl,

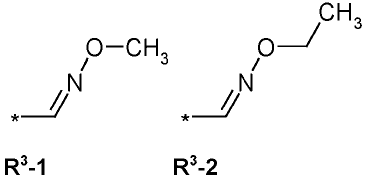

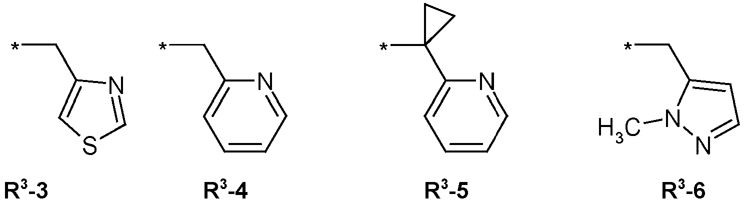

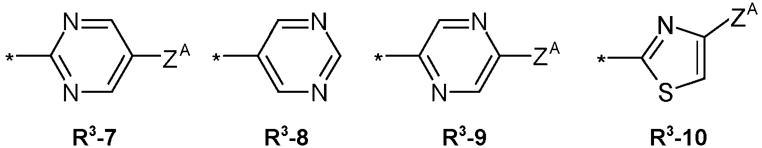

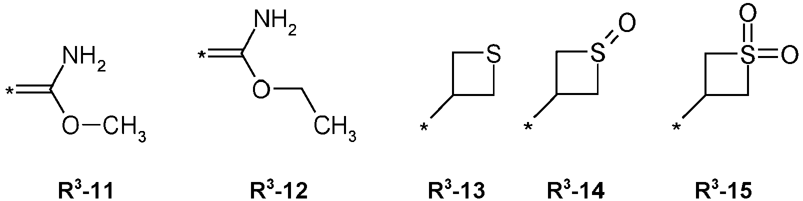

wherein Z^{A} = hydrogen, halogen, cyano, halomethyl (CF₃);
R⁴ = hydrogen, ethyl, methoxymethyl, halomethoxymethyl, ethoxymethyl, haloethoxymethyl, propoxymethyl, methylcarbonyl, ethylcarbonyl, propylcarbonyl, cyclopropylcarbonyl, methoxycarbonyl, methoxymethylcarbonyl, aminocarbonyl, ethylaminocarbonylmethyl, ethylaminocarbonylethyl, dimethoxyethyl, propynylaminocarbonylmethyl, haloethylaminocarbonylmethyl, cyanomethylaminocarbonylmethyl or haloethylaminocarbonylethyl; or
R³ and R⁴ together form a substituent selected from the group consisting of: 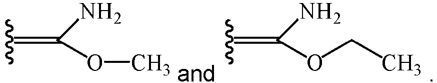 and/or
particles of a compound according to Formula (II)

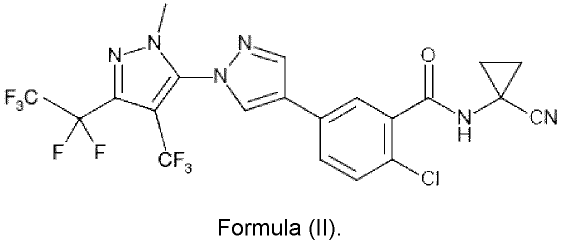

In a preferred embodiment of the invention and/or embodiments thereof, the isoxazoline compounds according to Formula (I) and/or the compound according to Formula (II) also include physiologically acceptable salts, esters, solvents and/or N-oxides thereof. In addition, the isoxazoline compounds according to Formula (I) and/or the compound according to Formula (II) refers equally to any of its polymorphic forms, enantiomers or stereoisomers thereof.

In a preferred embodiment of the invention and/or embodiments thereof, the isoxazoline compounds according to Formula (I) and/or the compound according to Formula (II) may exist in various isomeric forms. A reference to a compound according to Formula (I) and/or the compound according to Formula (II) includes all possible isomeric forms of such a compound.

In a preferred embodiment of the invention and/or embodiments thereof, the isoxazoline compounds according to Formula (I) and/or the compound according to Formula (II) may employ a racemic mixture of the isoxazoline compounds according to Formula (I) and/or the compound according to Formula (II), i.e. contain equal amounts of the enantiomers of the corresponding compound.

In an alternatively preferred embodiment of the invention and/or embodiments thereof, the isoxazoline compounds according to Formula (I) and/or the compound according to Formula (II) may employ isoxazoline compounds according to Formula (I) and/or the compound according to Formula (II) that contain enriched stereoisomers compared to the racemic mixture in one of the enantiomers of corresponding compound.

Also, the isoxazoline compounds according to Formula (I) and/or the compound according to Formula (II) may be an essentially pure stereoisomer of the corresponding compound. Especially preferred is the (S)-enantiomer of the isoxazoline compounds according to Formula (I) or the compound according to Formula (II). Such enriched or purified stereoisomer preparations of the isoxazoline compounds according to Formula (I) and/or the compound according to Formula (II) may be prepared by methods known in the art. For the chemical preparation of the products of the invention, a person skilled in the art is regarded as having at his disposal, inter alia, the entire contents of "Chemical Abstracts" and of the documents which are cited therein. Examples are chemical processes utilizing catalytic asymmetric synthesis or the separation of diastereomeric salts (see e.g.: WO 2009/063910 and JP 2011/051977, respectively).

In a preferred embodiment of the invention and/or embodiments thereof, the present composition comprises the isoxazoline compounds according to Formula (I) and/or the compound according to Formula (II) in an amount of about 0.1% by weight to about 50.0% by weight, preferably of about 1.0% by weight to about 45% by weight, more preferably of about 2.0 % by weight to about 35% by weight, even more preferably of about 3% by weight to about 25% by weight, in particular of about 5% by weight to about 15% by weight, especially about 9% by weight to 12% by weight, based on the total weight of the composition.

In a preferred embodiment of the invention and/or embodiments thereof, the present composition comprises the isoxazoline compounds according to Formula (I) and/or the compound according to Formula (II) in an amount of 0.1% by weight, about 0.25% by weight, about 0.5% by weight, about 1% by weight, about 2% by weight, about 3% by weight, about 4% by weight, about 5% by weight, about 6% by weight, about 7% by weight, about 8% by weight, about 9% by weight, about 10% by weight, about 11% by weight, about 12% by weight, about 13% by weight, about 14% by weight, about 15% by weight, about 20% by weight or about 25% by weight based on the weight of the composition.

In a preferred embodiment of the invention and/or embodiments thereof, in the isoxazoline compound according to Formula (I) T is selected from 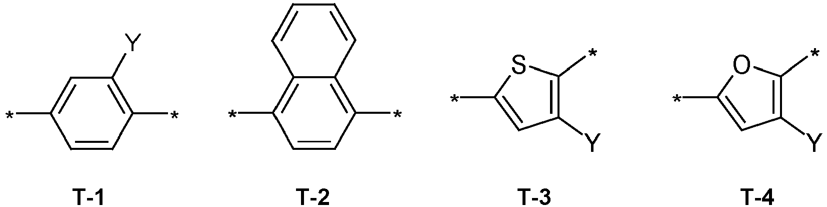 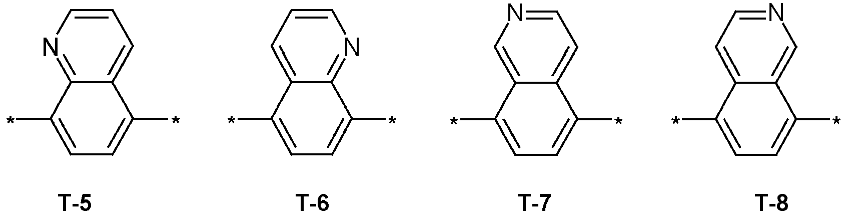 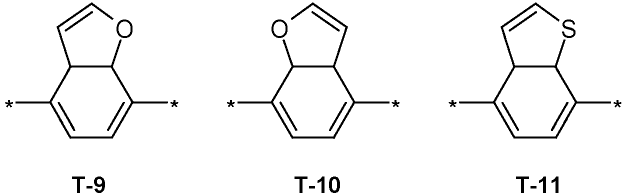 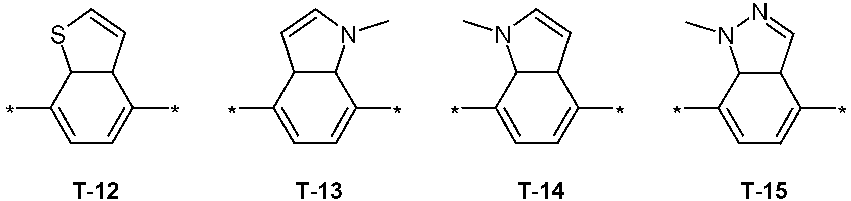 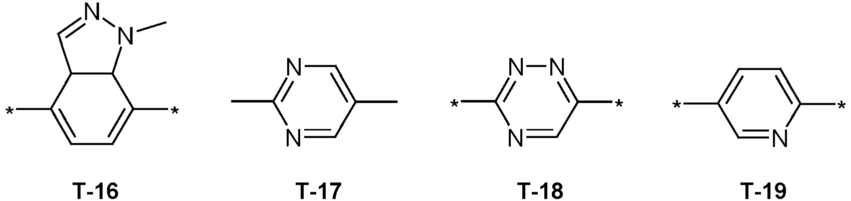 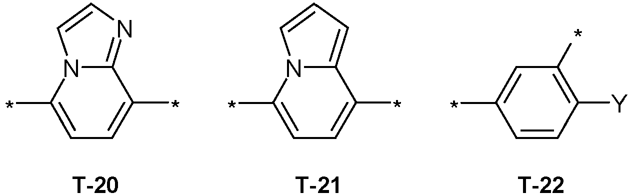 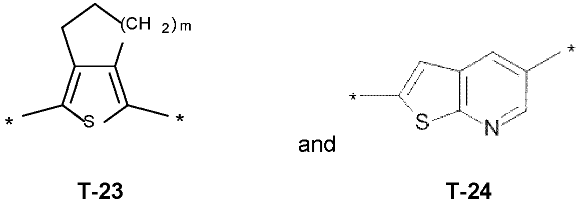 wherein in T-1, T-3 and T-4, the radical Y is preferably hydrogen, halogen, methyl, halomethyl, ethyl or haloethyl.

In a preferred embodiment of the invention and/or embodiments thereof, in the isoxazoline compound according to Formula (I) Q is selected from 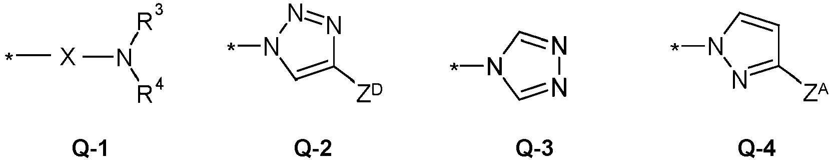 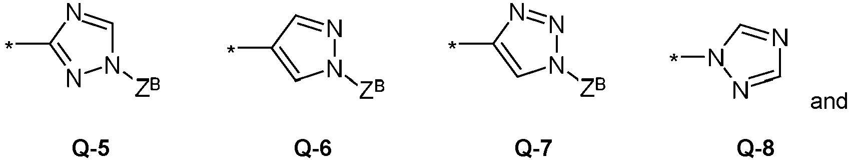 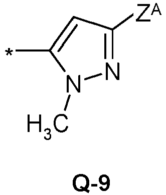
wherein R³, R⁴, X and Z^{A} are as defined above and
Z^{B} is 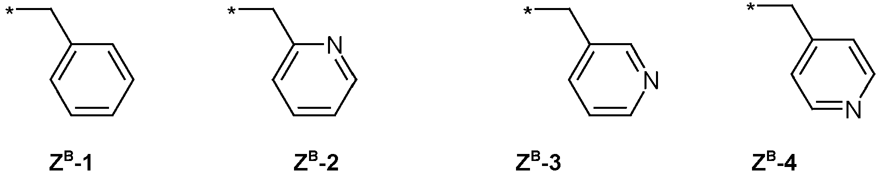 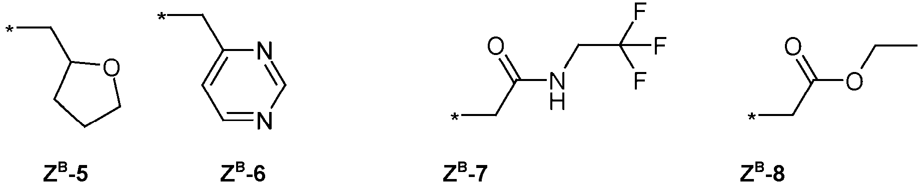 or 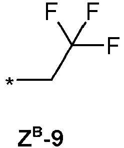
Z^{D} is 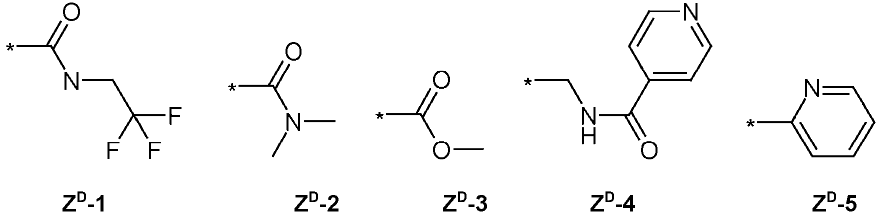 or

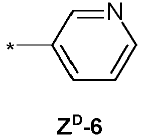

Preferred compounds of Formula (I) are listed in Table 1:

**Table 1:**

| **(R¹)ₙ** | **R²** | **R³** | **R⁴** | **T** | **Y** | **Q** | **Z** | **X** |
|---|---|---|---|---|---|---|---|---|
| 3-CI, 5Cl | CF₃ | CH₂CF₃ | H | T-2 | - | Q-1 | - | C(O) |
| 3-CI, 5Cl | CF₃ | CH₂CH₃ | H | T-2 | - | Q-1 | - | C(O) |
| 3-CI, 5Cl | CF₃ | CH₂CH₂OCH₃ | H | T-2 | - | Q-1 | - | C(O) |

| (continued) | | | | | | | | |
|---|---|---|---|---|---|---|---|---|
| **(R¹)ₙ** | **R²** | **R³** | **R⁴** | **T** | **Y** | **Q** | **Z** | **X** |
| 3-CI, 5Cl | CF₃ | CH₂C(O)NHCH₂CF₃ | H | T-2 | - | Q-1 | - | C(O) |
| 3-CI, 5Cl | CF₃ | CH₂C(O)NHCH₂CH₃ | H | T-2 | - | Q-1 | - | C(O) |
| 3-CF₃, 5-CF₃ | CF₃ | CH₂C(O)NHCH₂CF₃ | H | T-2 | - | Q-1 | - | C(O) |
| 3-CF₃, 5-CF₃ | CF₃ | CH₂C(O)NHCH₂CH₃ | H | T-2 | - | Q-1 | - | C(O) |
| 3-CF₃, 5-CI | CF₃ | CH₂C(O)NHCH₂CF₃ | H | T-2 | - | Q-1 | - | C(O) |
| 3-CF₃, 5-CI | CF₃ | CH₂C(O)NHCH₂CH₃ | H | T-2 | - | Q-1 | - | C(O) |
| 3-CI, 5Cl | CF₃ | - | | T-2 | - | Q-6 | Z^{B}-7 | |
| 3-CI, 5Cl | CF₃ | - | - | T-2 | - | Q-7 | Z^{B}-7 | |
| 3-CI, 5Cl | CF₃ | - | - | T-2 | - | Q-5 | Z^{B}-7 | |
| 3-CI, 5Cl | CF₃ | - | - | T-2 | - | Q-2 | Z^{D}-1 | |
| 3-CI, 5Cl | CF₃ | CH₂C(O)NHCH₂CF₃ | H | T-3 | CH₃ | Q-1 | - | C(O) |
| 3-CI, 5Cl | CF₃ | CH₂C(O)NHCH₂CC | H | T-3 | CH₃ | Q-1 | - | C(O) |
| 3-CI, 5Cl | CF₃ | CH₂C(O)NHCH₂CN | H | T-3 | CH₃ | Q-1 | - | C(O) |
| 3-CI, 5Cl | CF₃ | CH₂C(O)NHCH₂CH₃ | H | T-3 | CH₃ | Q-1 | - | C(O) |
| 3-CF₃, 5-CF₃ | CF₃ | CH₂C(O)NHCH₂CF₃ | H | T-3 | CH₃ | Q-1 | - | C(O) |
| 3-CF₃, 5-CF₃ | CF₃ | CH₂C(O)NHCH₂CH₃ | H | T-3 | CH₃ | Q-1 | - | C(O) |
| 3-CI, 4-CI, 5-CI | CF₃ | CH₂C(O)NHCH₂CF₃ | H | T-3 | CH₃ | Q-1 | - | C(O) |
| 3-CI, 4-CI, 5-CI | CF₃ | CH₂C(O)NHCH₂CH₃ | H | T-3 | CH₃ | Q-1 | - | C(O) |
| 3-CI, 4-F, 5-CI | CF₃ | CH₂C(O)NHCH₂CF₃ | H | T-3 | CH₃ | Q-1 | - | C(O) |
| 3-CI, 4-F, 5-CI | CF₃ | CH₂C(O)NHCH₂CH₃ | H | T-3 | CH₃ | Q-1 | - | C(O) |
| 3-CI, 5-CI | CF₃ | CH₂C(O)NHCH₂CF₃ | H | T-20 | - | Q-1 | - | C(O) |
| 3-CI, 5-CI | CF₃ | CH₂C(O)NHCH₂CH₃ | H | T-20 | - | Q-1 | - | C(O) |
| 3-CF₃, 5-CF₃ | CF₃ | CH₂C(O)NHCH₂CF₃ | CH₃ | T-20 | - | Q-1 | - | C(O) |
| 3-CF₃, 5-CF₃ | CF₃ | CH₂C(O)NHCH₂CH₃ | CH₃ | T-20 | - | Q-1 | - | C(O) |
| 3-CF₃, 5-CF₃ | CF₃ | CH₂C(O)NHCH₂CF₃ | H | T-20 | - | Q-1 | - | C(O) |
| 3-CF₃, 5-CF₃ | CF₃ | CH₂C(O)NHCH₂CH₃ | H | T-20 | - | Q-1 | - | C(O) |
| 3-CF₃, 5-CF₃ | CF₃ | CH₂C(O)NHCH₂CF₃ | H | T-21 | - | Q-1 | - | C(O) |
| 3-CF₃, 5-CF₃ | CF₃ | CH₂C(O)NHCH₂CH₃ | H | T-21 | - | Q-1 | - | C(O) |
| 3-CI, 5-CI | CF₃ | CH₂C(O)NHCH₂CF₃ | H | T-21 | - | Q-1 | - | C(O) |
| 3-CI, 5-CI | CF₃ | CH₂C(O)NHCH₂CH₃ | H | T-21 | - | Q-1 | - | C(O) |
| 3-CI, 5-CI | CF₃ | CH₂CH₂SCH₃ | H | T-21 | - | Q-1 | - | C(O) |
| 3-CI, 4-CI, 5-CI | CF₃ | C(O)CH₃ | H | T-22 | F | Q-1 | - | CH₂ |
| 3-CI, 4-CI, 5-CI | CF₃ | C(O)CH(CH₃)₂ | H | T-22 | F | Q-1 | - | CH₂ |
| 3-CI, 4-CI, 5-CI | CF₃ | C(O)-cyclo-propyl | H | T-22 | F | Q-1 | - | CH₂ |
| 3-CI, 4-F, 5-CI | CF₃ | C(O)CH₃ | H | T-22 | F | Q-1 | - | CH₂ |
| 3-CI, 4-CI, 5-CI | C₃ | C(O)CH₂CH₃ | H | T-22 | F | Q-1 | - | CH₂ |
| 3-CI, 4-F, 5-CI | CF₃ | C(O)CH₃ | H | T-22 | C! | Q-1 | - | CH₂ |
| 3-CI, 5-CI | CF₃ | CH₂C(O)NHCH₂CF₃ | H | T-1 | CH₃ | Q-1 | - | C(O) |
| 3-CI, 5-CI | CF₃ | CH₂C(O)NHCH₂CH₃ | H | T-1 | CH₃ | Q-1 | - | C(O) |
| 3-CI, 5-CI | CF₃ | R³-1 (Z) | H | T-1 | CH₃ | Q-1 | - | C(O) |
| 3-CI, 5-CI | CF₃ | R³-1 (E) | H | T-1 | CH₃ | Q-1 | - | C(O) |

More preferred compounds of Formula (I) are listed in Table 2.

**Table 2:**

| **(R¹)ₙ** | **R²** | **R³** | **R⁴** | **T** | **Y** | **Q** | **Z** | **X** |
|---|---|---|---|---|---|---|---|---|
| 3-CI, 5Cl | CF₃ | CH₂CF₃ | H | T-2 | - | Q-1 | - | C(O) |
| 3-CI, 5Cl | CF₃ | CH₂CH₃ | H | T-2 | - | Q-1 | - | C(O) |
| 3-CI, 5Cl | CF₃ | CH₂CH₂OCH₃ | H | T-2 | - | Q-1 | - | C(O) |
| 3-CI, 5Cl | CF₃ | CH₂C(O)NHCH₂CF₃ | H | T-2 | - | Q-1 | - | C(O) |
| 3-CF₃, 5-CF₃ | CF₃ | CH₂C(O)NHCH₂CF₃ | H | T-2 | - | Q-1 | - | C(O) |
| 3-CF₃, 5-Cl | CF₃ | CH₂C(O)NHCH₂CF₃ | H | T-2 | - | Q-1 | - | C(O) |
| 3-CI, 5Cl | CF₃ | - | | T-2 | - | Q-6 | Z^{B}-7 | |
| 3-CI, 5Cl | CF₃ | - | - | T-2 | - | Q-7 | Z^{B}-7 | |
| 3-CI, 5Cl | CF₃ | - | - | T-2 | - | Q-5 | Z^{B}-7 | |
| 3-CI, 5Cl | CF₃ | - | - | T-2 | - | Q-2 | Z^{D}-1 | |
| 3-CI, 5Cl | CF₃ | CH₂C(O)NHCH₂CF₃ | H | T-3 | CH₃ | Q-1 | - | C(O) |
| 3-CI, 5Cl | CF₃ | CH₂C(O)NHCH₂CC | H | T-3 | CH₃ | Q-1 | - | C(O) |
| 3-CI, 5Cl | CF₃ | CH₂C(O)NHCH₂CN | H | T-3 | CH₃ | Q-1 | - | C(O) |
| 3-CF₃, 5-CF₃ | CF₃ | CH₂C(O)NHCH₂CF₃ | H | T-3 | CH₃ | Q-1 | - | C(O) |
| 3-CI, 4-CI, ⁵⁻ Cl | CF₃ | CH₂C(O)NHCH₂CF₃ | H | T-3 | CH₃ | Q-1 | - | C(O) |
| 3-CI, 4-F, 5-CI | CF₃ | CH₂C(O)NHCH₂CF₃ | H | T-3 | CH₃ | Q-1 | - | C(O) |
| 3-CI, 5-CI | CF₃ | CH₂C(O)NHCH₂CF₃ | H | T-20 | - | Q-1 | - | C(O) |
| 3-CF₃, 5-CF₃ | CF₃ | CH₂C(O)NHCH₂CF₃ | CH₃ | T-20 | - | Q-1 | - | C(O) |
| 3-CF₃, 5-CF₃ | CF₃ | CH₂C(O)NHCH₂CF₃ | H | T-20 | - | Q-1 | - | C(O) |
| 3-CF₃, 5-CF₃ | CF₃ | CH₂C(O)NHCH₂CF₃ | H | T-21 | - | Q-1 | - | C(O) |
| 3-CI, 5-CI | CF₃ | CH₂C(O)NHCH₂CF₃ | H | T-21 | - | Q-1 | - | C(O) |
| 3-CI, 5-CI | CF₃ | CH₂CH₂SCH₃ | H | T-21 | - | Q-1 | - | C(O) |
| 3-CI, 4-CI, Cl | 5-CF₃ | C(O)CH₃ | H | T-22 | F | Q-1 | - | CH₂ |
| 3-CI, 4-CI, ⁵⁻ Cl | CF₃ | C(O)CH(CH₃)₂ | H | T-22 | F | Q-1 | - | CH₂ |
| 3-CI, 4-CI, ⁵⁻ Cl | CF₃ | C(O)-cyclopropyl | H | T-22 | F | Q-1 | - | CH₂ |
| 3-CI, 4-F, 5-CI | CF₃ | C(O)CH₃ | H | T-22 | F | Q-1 | - | CH₂ |
| 3-CI, 4-CI, ⁵⁻ CI | CF₃ | C(O)CH₂CH₃ | H | T-22 | F | Q-1 | - | CH₂ |
| 3-CI, 4-F, 5-CI | CF₃ | C(O)CH₃ | H | T-22 | C! | Q-1 | - | CH₂ |
| 3-CI, 5-CI | CF₃ | CH₂C(O)NHCH₂CF₃ | H | T-1 | CH₃ | Q-1 | - | C(O) |
| 3-I, 5-CI | CF₃ | R³-1 (Z) | H | T-1 | CH₃ | Q-1 | - | C(O) |
| 3-CI, 5-CI | CF₃ | R³-1 (E) | H | T-1 | CH₃ | Q-1 | - | C(O) |

In a particularly preferred embodiment of the invention and/or embodiments thereof, the isoxazoline compound is represented by Formula (Ia) 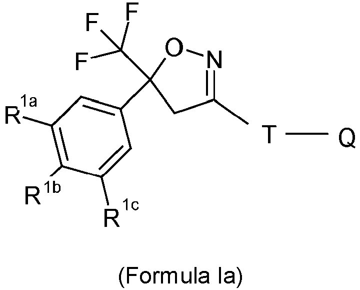 wherein
R^{1a}, R^{1b}, R^{1c} are independently from each other hydrogen, Cl or CF₃, more preferably R^{1a} and R^{1c} are Cl or CF₃, and R^{1b} is hydrogen,
T is 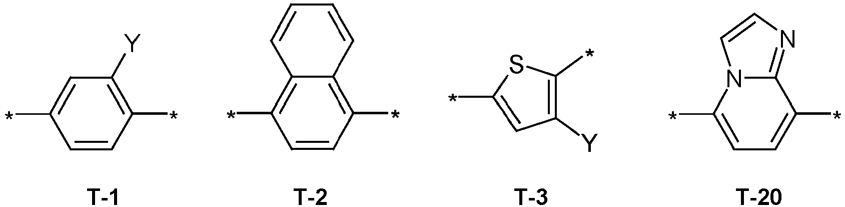 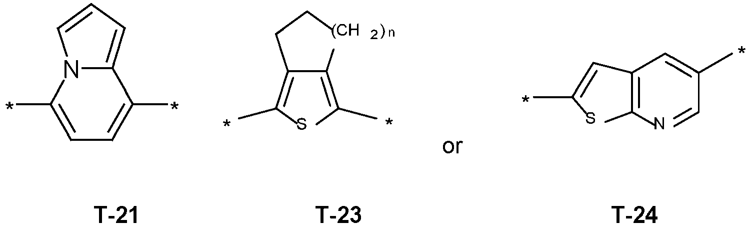
wherein Y is methyl, Cl, Br, F, CN or C(S)NH₂ and
Q is as described above.

In another preferred embodiment of the invention and/or embodiments thereof R³ is H and R⁴ is -CH₂-C(O)-NH-CH₂-CF₃, -CH₂-C(O)-NH-CH₂-CH₃, -CH₂-CH₂-CF₃ or -CH₂-CF₃.

In an embodiment of the invention and/or embodiments thereof, the isoxazoline compound of Formula (I) is 4-[5-(3,5-dichlorophenyl)-5-trifluoromethyl-4,5-dihydroisoxazol-3-yl]-2-methyl-N-[(2,2,2-trifluoro-ethylcarbamoyl)-methyl]-benzamide (CAS RN [864731-61-3]; USAN fluralaner).

In another embodiment of the invention and/or embodiments thereof, the isoxazoline compound of Formula (I) is (Z)-4-[5-(3,5-dichlorophenyl)-5-trifluoromethyl-4,5-dihydroisoxazol-3-yl]-N-[(methoxyimino)methyl]-2-methylbenzamide (CAS RN [928789-76-8]).

In an embodiment of the invention and/or embodiments thereof, the isoxazoline compound of Formula (I) is 4-[5-[3-chloro-5-(trifluoromethyl)phenyl]-4,5-dihydro-5-(trifluoromethyl)-3-isoxazolyl]-N-[2-oxo-2-[(2,2,2-trifluoroethyl)amino]ethyl]-1-naphthalenecarboxamide (CAS RN 1093861-60-9, USAN afoxolaner) that was disclosed in WO 2007/079162.

In an embodiment of the invention and/or embodiments thereof, the isoxazoline compound of Formula (I) is lotilaner (CAS RN: 1369852-71-0; 3-methyl-N-[2-oxo-2-(2,2,2-trifluoroethyl-amino)ethyl]-5-[(5S)-5-(3,4,5-trichlorophenyl)-5-(trifluoromethyl)-4H-1,2-oxazol-3-yl]thiophene-2-carboxamide).

In an embodiment of the invention and/or embodiments thereof, the isoxazoline compound of Formula (I) is sarolaner (CAS RN: 1398609-39-6; 1-(5'-((5S)-5-(3,5-dichloro-4-fluorophenyl)-5-(trifluoromethyl)-4,5-dihydroisoxazol-3-yl)-3'-H-spiro(azetidine-3,1'-(2) benzofuran)-1-yl)-2-(methylsulfonyl) ethanone).

In an embodiment of the invention and/or embodiments thereof, the isoxazoline compound of Formula (I) is 5-[5-(3,5-dichlorophenyl)-4,5-dihydro-5-(trifluoromethyl)-3-isoxazolyl]-3-methyl-N-[2-oxo-2-[(2,2,2-trifluoroethyl)amino]ethyl]- 2-thiophenecarboxamide (CAS RN 1231754-09-8) that was disclosed in WO 2010/070068.

In an embodiment of the invention and/or embodiments thereof, the isoxazoline compound of Formula (I) is 4-[5-(3,5-dichlorophenyl)-5-(trifluoromethyl)-4H-isoxazol-3-yl]-2-methyl-N-(thietan-3-yl)benzamide (CAS RN 1164267-94-0) that was disclosed in WO 2009/0080250.

In an alternative embodiment of the invention and/or embodiments thereof, compound b) comprises particles of the compound according to Formula (II). The compound of Formula (II) is 2-chloro-*N*-(1-cyanocyclopropyl)-5-[1'-methyl-3'-(1,1,2,2,2-pentafluoroethyl)-4'-(trifluoromethyl)[1,5'-bi-1*H*-pyrazol]-4-yl]benzamide; Tigolaner (CAS RN 1621436-41-6) that was disclosed in WO 2019/012377.

In an embodiment of the invention and/or embodiments thereof, the isoxazoline compound of Formula (I) is selected from the group consisting of fluralaner, afoxolaner, lotilaner, sarolaner and mixtures thereof.

In an embodiment of the invention and/or embodiments thereof, the isoxazoline compound of Formula (I) is afoxolaner or fluralaner.

In a preferred embodiment of the invention and/or embodiments thereof, the present composition comprises afoxolaner or fluralaner, preferably fluralaner, in an amount of about 0.1% by weight to about 50.0% by weight, preferably of about 1.0% by weight to about 45% by weight, more preferably of about 2.0 % by weight to about 35% by weight, even more preferably of about 3% by weight to about 25% by weight, in particular of about 5% by weight to about 15% by weight, especially about 9% by weight to 12% by weight, based on the total weight of the composition.

In an embodiment of the invention and/or embodiments thereof, the isoxazoline compound of Formula (I)) is the (*S*)-enantiomer of afoxolaner (esafoxolaner) or the (*S*)-enantiomer of fluralaner.

In an embodiment of the invention and/or embodiments thereof, the isoxazoline compound of Formula (I) is the (*S*)-enantiomer of fluralaner.

In one embodiment the composition according to the invention comprises particles of a compound according to Formula (II)

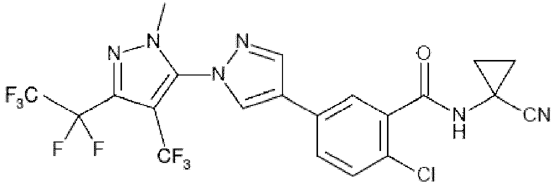

It has been found that the inventive injectable compositions comprising particles of the isoxazoline compound according to Formula (I) and/or the compound according to Formula (II) with a defined particle size have especially beneficial properties.

In one embodiment of the invention and/or embodiments thereof the volume weighted particle size of the microspheres (a) comprising (a1) one or more physiologically active lactone(s), preferably moxidectin, and polymer (a2), preferably polycaprolactone, and the volume weighted particle size of the particle of the isoxazoline compound according to Formula (I) or the compound according to Formula (II) are in a similar range.

In one embodiment of the invention and/or embodiments thereof the volume weighted particle size of the microspheres (a) comprising (a1) one or more physiologically active lactone(s), preferably moxidectin, and (a2), y polycaprolactone, and the volume weighted particle size of the particle of the isoxazoline compound according to Formula (I) or the compound according to Formula (II) are not in a similar range.

The volume weighted particle size of the microspheres (a) and the volume weighted particle size of particles of the isoxazoline compound according to Formula (I) and/or the compound according to Formula (II) are measured by the same methods.

In an embodiment of the invention or embodiments thereof, the D10 of the volume weighted particle size distribution of particles of isoxazoline compound according to Formula (I) and/or compound according to Formula (II) is from about 5 µm to about 75 µm, more preferably from about 10 µm to about 50 µm, even more preferably from about 15 µm to about 40 µm, in particular from about 20 µm to about 35 µm.

In an embodiment of the invention or embodiments thereof, the D50 of the volume weighted particle size distribution of the particles of isoxazoline compound according to Formula (I) and/or compound according to Formula (II) is from about 65 µm to about 150 µm, more preferably from about 80 µm to about 120 µm, even more preferably from about 85 µm to about 115 µm, in particular from about 90 µm to about 105 µm.

In an embodiment of the invention or embodiments thereof, the D90 of the volume weighted particle size distribution of the particles of isoxazoline compound according to Formula (I) and/or compound according to Formula (II) is from about 100 µm to about 250 µm, more preferably from about 130 µm to about 200 µm, even more preferably from about 150 µm to about 180 µm, in particular from about 155 µm to about 175 µm.

The microspheres (a) and particles (b) are suspended in an aqueous carrier comprising one or more suspending agents and/or one or more wetting agents.

An aqueous carrier is as a liquid aqueous or inert medium. An aqueous carrier can be used as solvent (or diluent) in which the active agent is formulated and or administered.

In a preferred embodiment of the invention and/or embodiments thereof an aqueous carrier is water, or water and water miscible liquid.

A water miscible liquid (also referred to as a cosolvent) can be, but is not limited to, ethanol, isopropanol, benzyl alcohol, glycol ethers (e.g. including, but limited to, diethylene glycol mono-ethylether (DGME, Transcutol^{®}), butyl diglycol, dipropylene glycol n-butyl ether, ethylene glycol monoethyl ether, ethylene glycol monomethyl ether, dipropylene glycol monomethyl ether, propylene glycol monomethyl ether, propylene glycol monoethyl ether, and the like), liquid polyethylene glycols (PEGs) (for example, PEG 400), propylene glycol, carbonates (e.g., propylene carbonate), cyclic ethers (e.g. as tetrahydrofuran and dioxane), 2-pyrrolidone, N-methylpyrrolidone, dimethyl isosorbide (DMI), dimethylformamide, acetamide dimethylacetamide, dimethyl sulfoxide, glycerol or a mixture thereof.

In one embodiment of the invention and/or embodiments thereof, the water miscible liquid can be a polar protic solvent including, but not limited to, an alcohol such as ethanol, isopropanol or a glycol or glycol ether.

In one embodiment of the invention and/or embodiments thereof, the water miscible liquid can be a polar aprotic solvent such as N-methylpyrrolidone, dimethyl isosorbide, dimethylacetamide, dimethyl sulfoxide or propylene carbonate.

In an embodiment, the pharmaceutical composition is substantially free of a water miscible liquid (co-solvent).

In an embodiment of the invention and/or embodiments thereof, the aqueous carrier comprises a suspending agent. Herein, a suspending agent can be considered as a substance that can be added to a fluid to promote particle suspension or dispersion and reduce sedimentation.

Suspending agents include, but are not limited to sodium carboxymethylcellulose, methylcellulose, hydroxypropyl methylcellulose, sodium alginate, polyvinylpyrrolidone, gum tragacanth, gum acacia and mixtures thereof.

In an embodiment of the invention and/or embodiments thereof, the suspending agent is selected from carboxy methyl cellulose, in particular sodium carboxy methyl cellulose (NaCMC), polyvinylpyrrolidone, methylcellulose and mixtures thereof.

In an embodiment of the invention and/or embodiments thereof, the suspending agent is carboxy methyl cellulose, in particular sodium carboxy methyl cellulose (NaCMC).

In an embodiment of the invention and/or embodiments thereof, the suspending agent is polyvinylpyrrolidone.

In an embodiment of the invention and/or embodiments thereof, the suspending agent is methylcellulose.

In an embodiment of the invention and/or embodiments thereof, the present composition comprises a suspending agent in an amount of about 0.5% by weight to about 15 % by weight, preferably about 1.0% by weight to about 12.5% by weight, more preferably about 1.5% by weight to about 10% by weight about 2.0% by weight, in particular to about 7.5% by weight, based on the total weight of the composition.

In an embodiment of the invention and/or embodiments thereof, the aqueous carrier comprises a wetting agent.

Wetting agents (sometimes also referred to as dispersing agents) are compounds suitable to lower the surface tension between phases such a liquid-liquid, liquid-gas and liquid-solid.

Wetting agents include, but are not limited to naturally-occurring phosphatides such as lecithin, condensation products of an alkylene oxide with fatty acids such as polyoxyethylene stearate, condensation products of ethylene oxide with long chain aliphatic alcohols such as heptadeca-ethyleneoxycetanol, condensation products of ethylene oxide with partial esters derived from fatty acids and a hexitol such as polyoxyethylene sorbitol monooleate, or condensation products of ethylene oxide with partial esters derived from fatty acids, hexitol anhydrides such as polyethylene sorbitan monooleate, sorbitan fatty acid esters (Spans), polyoxyethylene sorbitan fatty acid esters (polysorbates/Tweens), polyoxyethylene castor oil derivatives (Cremaphors), and TPGS (d-α-Tocopheryl polyethylene glycol 1000 succinate).

In an embodiment of the invention and/or embodiments thereof, the wetting agent is poloxamer.

Poloxamers are nonionic triblock copolymers comprising a central hydrophobic chain of polyoxypropylene (polypropylene oxide)) flanked by two hydrophilic chains of polyoxyethylene (poly(ethylene oxide)). Poloxamer 124 (also known as Lutrol L44 or Kollisolv P124) is poly(ethylene glycol)-block-poly(propylene glycol)-block-poly(ethylene glycol). Poloxamer 188 also known as Lutrol F68 or Kolliphor P188is another polyethylene glycol)-block-poly(propylene glycol)-block-poly(ethylene glycol).

In an embodiment of the invention and/or embodiments thereof, the present composition comprises wetting agent in an amount of about 0.01% by weight to about 1.0% by weight, preferably about 0.05% by weight to about 0.9% by weight, more preferably about 0.1% by weight to about 0.8% by weight, in particular about 0.2% by weight to about 0.5% by weight, based on the total weight of the composition.

In one preferred embodiment of the invention and/or embodiments thereof, the present composition further comprises one or more physiologically acceptable excipients. Physiologically acceptable excipients are known in the art. For example, they are described in "Gennaro, Remington: The Science and Practice of Pharmacy" (20th Edition, 2000). All such physiologically acceptable excipients must be substantially physiologically or veterinary pure and non-toxic in the amounts employed and must be compatible with the active ingredients.

In one preferred embodiment of the invention and/or embodiments thereof, the physiologically acceptable excipients are contained in the microspheres (a) and/or in the aqueous carrier.

In one preferred embodiment of the invention and/or embodiments, thereof the physiological acceptable excipients are selected from surfactants, buffers, preservatives and mixtures thereof.

Surfactants can be regarded as substances lowering the interfacial tension between two phases. Common surfactants are alkylsulfates (for example sodium lauryl sulfate), alkyltrimethylammonium salts, alcohol ethoxylates and the like.

Again, these compounds, as well as their amounts are well known in the art.

In another embodiment of the invention and/or embodiments thereof, the inventive compositions may include polyoxyl 35 castor oil (Kolliphor^{®} EL) as a surfactant. In other embodiments, the inventive compositions may include polyoxyl 40 hydrogenated castor oil (Kolliphor^{®} RH 40) or polyoxyl 60 hydrogenated castor oil as surfactant. The compositions of the invention may also include a combination of surfactants.

It is preferred that the surfactant is present in the aqueous carrier.

Buffers are substances to maintain/adjust the pH value of a product. Again, such compounds are well known to a practitioner in the art as well as how to use these compounds. Buffering systems include, but are not limited to, for example systems selected from the group consisting of acetic acid/acetate, malic acid/malate, citric acid/citrate, tartaric acid/tartrate, lactic acid/lactate, phosphoric acid/phosphate, phosphoric acid/hydrogen phosphate, glycine/glycimate, lutamic acid/glutamates and sodium carbonate/hydrogen carbonate, especially phosphoric acid/sodium phosphate or citric acid/sodium citrate. It is preferred that the buffer is present in the aqueous carrier.

A preservative is a substance that is added to a mixture, in particular to a mixture containing a physiologically active agent to prevent decomposition by microbial growth or by undesirable chemical changes. Preferred is benzyl alcohol. The preservative is present in the aqueous carrier or in the microspheres (a).

In an embodiment of the invention and/or embodiments thereof, the composition comprises eprinomectin as (a1) physiologically active macrocyclic lactone and fluralaner, preferably (S)-fluralaner, as (b) the isoxazoline compound of Formula (I).

In an embodiment of the invention and/or embodiments thereof, the composition comprises milbemycin oxime as (a1) physiologically active macrocyclic lactone and fluralaner, preferably (S)-fluralaner, as (b) isoxazoline compound of Formula (I).

In an embodiment of the invention and/or embodiments thereof, the composition comprises selamectin as (a1) physiologically active macrocyclic lactone and fluralaner, preferably (S)-fluralaner, as (b) isoxazoline compound of Formula (I).

In an especially preferred embodiment of the invention and/or embodiments thereof, the composition comprises moxidectin as (a1) physiologically active macrocyclic lactone and fluralaner, preferably (S)-fluralaner, as (b) isoxazoline compound of Formula (I).

In an embodiment of the invention and/or embodiments thereof, the composition comprises moxidectin in an amount of about 0.01% by weight to about 1.0% by weight, more preferably of about 0.05% by weight to about 0.85% by weight, even more preferably of about 0.1% by weight to about 0.7% by weight, in particular preferably of about 0.15% by weight to about 0.5% by weight, especially about 0.17% by weight and fluralaner in amount of about 0.1% by weight to about 50.0% by weight, preferably of about 1.0% by weight to about 45% by weight, more preferably of about 2.0 % by weight to about 35% by weight, even more preferably of about 3% by weight to about 25% by weight, in particular of about 5% by weight to about 15% by weight, especially about 9% by weight to 12% by weight, based on the total weight of the composition.

In an embodiment of the invention and/or embodiments thereof, the composition comprises eprinomectin as (a1) physiologically active macrocyclic lactone and afoxolaner, preferably (S)-afoxolaner, as (b) isoxazoline compound of Formula (I).

In an embodiment of the invention and/or embodiments thereof, the composition comprises milbemycin oxime as (a1) physiologically active macrocyclic lactone and afoxolaner, preferably (S)-afoxolaner, as (b) isoxazoline compound of Formula (I).

In an embodiment of the invention and/or embodiments thereof, the composition comprises selamectin as (a1) physiologically active macrocyclic lactone and afoxolaner, preferably (S)-afoxolaner, as (b) isoxazoline compound of Formula (I).

In an embodiment of the invention and/or embodiments thereof, the composition comprises moxidectin as (a1) physiologically active macrocyclic lactone and afoxolaner, preferably (S)-afoxolaner, as (b) isoxazoline compound of Formula (I).

In an embodiment of the invention and/or embodiments thereof, the composition comprises eprinomectin as (a1) physiologically active macrocyclic lactone and sarolaner as (b) isoxazoline compound of Formula (I).

In an embodiment of the invention and/or embodiments thereof, the composition comprises milbemycin oxime as (a1) physiologically active macrocyclic lactone and sarolaner as (b) isoxazoline compound of Formula (I).

In an embodiment of the invention and/or embodiments thereof, the composition comprises selamectin as (a1) physiologically active macrocyclic lactone and sarolaner as (b) isoxazoline compound of Formula (I).

In an embodiment of the invention and/or embodiments thereof, the composition comprises moxidectin as (a1) physiologically active macrocyclic lactone and sarolaner as (b) isoxazoline compound of Formula (I).

In an embodiment of the invention and/or embodiments thereof, the composition comprises eprinomectin as (a1) physiologically active macrocyclic lactone and lotilaner as (b) isoxazoline compound of Formula (I).

In an embodiment of the invention and/or embodiments thereof, the composition comprises milbemycin oxime as (a1) physiologically active macrocyclic lactone and lotilaner as (b) isoxazoline compound of Formula (I).

In an embodiment of the invention and/or embodiments thereof, the composition comprises selamectin as (a1) physiologically active macrocyclic lactone and lotilaner as (b) isoxazoline compound of Formula (I).

In an embodiment of the invention and/or embodiments thereof, the composition comprises moxidectin as (a1) physiologically active macrocyclic lactone and lotilaner as (b) isoxazoline compound of Formula (I).

Injectable compositions generally need to be sterilized prior to administration to an animal. In a preferred embodiment of the invention and/or embodiments thereof, the microspheres are sterilized, for example with gamma radiation or electron beam irradiation. Though physiologically active macrocyclic lactones are reported to degrade and lose much of the biological activity when irradiated, the microspheres (a) can be sterilized for injection by irradiation without the stability of the active ingredients being negatively impacted.

In an embodiment of the invention and/or embodiments thereof, the present composition can contain an additional therapeutic agent.

In an embodiment of the invention and/or embodiments thereof, the present composition can be administered in combination with an additional therapeutic agent. The administration of the additional therapeutic agent may be in the same composition or in separate compositions.

The additional therapeutic agent can preferably be a parasiticide or a vaccine, preferably another parasiticide.

The additional therapeutic agent may be selected from the group consisting of pro-benzimidazoles (e.g. febantel, netobimin, and thiophanate); benzimidazole derivatives, such as a thiazole benzimidazole derivatives (e.g. thiabendazole and cambendazole), carbamate benzimidazole derivatives (e.g. fenbendazole, albendazole (oxide), mebendazole, oxfendazole, parbendazole, oxibendazole, flubendazole and triclabendazole); imidazothiazoles (e.g. levamisole and tetramisole); tetrahydropyrimidine (e.g. morantel and pyrantel), salicylanilides (e.g. closantel, oxyclozanide, rafoxanide, and niclosamide); nitrophenolic compounds (e.g. nitroxynil and nitroscanate); benzenedisulfonamides (e.g. clorsulon); pyrazinoisoquinolines (e.g. praziquantel and epsiprantel); cyclooctadepsipeptides (e.g. emodepside); paraherquamides (e.g. derquantel) and amino-acetonitrile compounds (e.g. monepantel, AAD 1566); amidine compounds (e.g. amidantel and tribendimidinand mixtures thereof.

In one embodiment of the invention and/or embodiments thereof, the injectable veterinary pharmaceutical composition must be reconstituted prior to injection. For example, a mixture of (a) the microspheres and (b) particles of the isoxazoline compound(s) according to Formula (I) and/or the compound according to Formula (II) can be reconstituted in an aqueous carrier prior to injection.

Reconstitutable formulation is a formulation where a liquid vehicle (e.g. aqueous carrier) is in one container (e.g. vial) and one or more solid active ingredients (e.g. microspheres and particles) in another container and the content of the two containers are combined to form a liquid final formulation at some point prior to administration to the animal. In case of the composition of the current invention an aqueous suspension is formed that includes the macrocyclic lactone microspheres and the fluralaner particles in particulate form.

In another embodiment of the invention and/or embodiments thereof, the injectable veterinary composition is a ready to use composition that ready for injection to the animal.

It turned out, that the present invention provides injectable compositions comprising particles of isoxazoline compound(s) according to Formula (I) and/or the compound according to Formula (II) as well as microspheres comprising one or more physiologically active macrocyclic lactone(s) such as moxidectin, wherein the compositions provide safety, physical and chemical stability of the composition and the active agents and/or reduced risk of injection site irritation.

Physical stability of an injectable suspension is especially important to allow correct dosing by injecting a homogeneous suspension comprising the correct amount of both the one or more physiologically active macrocyclic lactone(s) and the isoxazoline compound according to Formula (I) and/or the compound according to Formula (II) and in one common formulation. In the present case, the inventors overcame the issue that the provision of a homogeneous suspension of two different solid components is difficult due to their different densities.

Further, it is also ensured that a stable composition is provided. In particular, stability with regard to the one or more physiologically active macrocyclic lactone(s), in particular moxidectin, which is especially challenging with regard to stable compositions, is provided.

Furthermore, a stable suspension is provided that can be easily re-suspended with the aqueous carrier by gentle shaking without causing foaming or floating or settling of the suspended particles, which negatively impacts the precision of the dosing. Furthermore, the final composition to be injected remains physically (and chemically) stable for the whole in-use period after re-suspension/reconstitution.

The microspheres (a) and the isoxazoline particles (b) and the present injectable veterinary pharmaceutical composition can preferably be sterilized by gamma radiation or electron beam without significant degradation, i.e. to maintain shelf life without significant loss of biological activity.

### Manufacturing of the injectable veterinary composition

Another aspect of the invention is a method of preparing the injectable veterinary composition according to the present invention comprising the steps of:
i) preparing particles of the isoxazoline compound of Formula (I) and/or a compound of Formula (II),
ii) preparing microspheres (a) by solvent evaporation, spinning disk atomization or spray drying and sieving the resulting product,
iii) preparing an aqueous carrier by dissolving one or more suspending agents and/or one or more wetting agents and water,
iv) adding the particles obtained from step i) and the microspheres obtained from step ii) to the aqueous carrier or vice versa.

As far as the components such as for example the physiologically active macrocyclic lactone (a1), the isoxazoline compound of Formula (I) and/or the suspending agent, their content and properties are concerned, the same as described above applies.

### Particles of isoxazoline compounds of Formula (I) and/or compound of Formula (II)

In step i) particles of the isoxazoline compound of Formula (I) and/or a compound of Formula (II) are prepared. The particles can for example be prepared according to the method as described in WO 2019/091940 A1.

In a preferred embodiment of the invention and/or embodiments thereof said process comprises initiating crystallization and then maintaining the temperature of the crystallization in the metastable region by removing, reheating and recycling a portion of the solvent thereby allowing the existing crystals to grow larger while minimizing the formation of newer smaller crystals.

The solvent includes, but is not limited to, methanol, ethanol, isopropanol (isopropyl alcohol), acetone, an ethyl acetate, acetonitrile, dimethyl acetamide (DMA), N-methylpyrrolidone (NMP), dimethyl sulfoxide (DMSO), N,N-diethy-m-toluamide (DEET), 2-pyrrolidone, g-hexalactone, glycofurol (tetraglycol), methyl ethyl ketone, diethylene glycol monoethyl ether (Transcutol^{®}), dimethylisosorbide, macrogol glycerol caprylcaprate (Labrasol^{®}), dipropylene glycol monomethyl ether (Dowanol^{™} DPM), glycerol formal, benzyl alcohol, methanol, polyethylene glycol 200, propylene carbonate, 1-methoxy-2-propyl acetate (Dowanol^{™} PMA), isopropylidene glycerol (solketal), ethyl alcohol, glycerol triacetate (triacetin), propylene glycol, triglycerides medium chain (Miglyol^{®} 812), ethyl oleate, toluene or mixtures thereof, preferably isopropanol (isopropyl alcohol).

In a preferred embodiment of the invention and/or embodiments thereof step b) of the process can be carried out at a temperature of 60°C to 75°C, preferably at about 65°C.

Alternatively, particles can be microspheres, such as e.g. PLGA microspheres.

### Macrocyclic lactone microspheres

In step ii) microspheres (a) are prepared by solvent evaporation, spinning disk atomization or spray drying and, optionally, sieving the resulting product.

Generally, solvent evaporation, spinning disk atomization, spray drying as well as sieving are methods known to skilled person.

Solvent evaporation is one of the most commonly use method to prepare microspheres. As is commonly done, the polymer is dissolved in a volatile organic solvent into which the drug is dissolved. The resultant solution is then added to the aqueous phase containing surfactant under high homogenization to form an emulsion. Finally, the organic solvent is evaporated either by increasing the temperature under reduced pressure or by continuous stirring yielding dispersion of droplets.

Spinning disk atomization - encapsulation technique that uses mechanical energy to pressurize the liquid film or increase it kinetic energy for possible disintegration in the form of droplets.

Spray drying is a method of producing dry powder from a liquid or slurry by rapidly drying with a hot gas.

In other words, the microspheres (a) can be regarded as microspheres prepared by incorporating the physiologically active macrocyclic lactone (a1) and optionally other excipients with a polymer (a2) and then forming microspheres of the resulting mixture by a variety of techniques such as the ones indicated above. Alternatively, the mixture of the physiologically active macrocyclic lactone (a1) and optionally other excipients with a polymer (a2) may be cooled to give a solid which may then be processed by procedures such as milling, grinding and the like.

In a preferred embodiment of the invention and or embodiments thereof, the microspheres (a) are prepared by solvent evaporation and optionally sieving the resulting product.

For this, it is preferred that the physiologically active macrocyclic lactone (a1), polymer (a2) and optionally further physiologically acceptable excipient can be dissolved in a solvent, preferably dichloromethane, and added, preferably to an aqueous solution, preferably polyvinyl alcohol in water. It is preferred that the above adding in step ii) is conducted at a temperature from about 20°C to about 30°C, preferably about 23°C to obtain a mixture. It is preferred that the above adding in step ii) is carried out under mechanical treatment such as stirring. Further, step ii) preferably comprises isolating the microspheres (a) by sieving the obtained mixture over a mesh, preferably a 75 µm sieve and subsequently drying, preferably at 23°C and/or reduced pressure.

In another preferred embodiment of the invention and or embodiments thereof the microspheres
a) are prepared by spinning disk and sieving the resulting product.

For this, it is preferred that the physiologically active macrocyclic lactone (a1), polymer (a2) and optionally further physiologically acceptable excipient can be dissolved in a solvent, preferably dichloromethane, and fed to a custom disc spinning with the standard setups known to the skilled person. It is preferred that the above feeding is conducted at a temperature of about 0°C to about 20°C. The resulting particles are preferably sieved over at least one mesh, preferably a 420 µm and a 250 µm sieve to obtain the microgranules (a). It turned out that "spinning disk" is a production technique for generating uniform spherical particles with a low particle size distribution span, for example through control of the process parameters such as melt temperature, flow rate and disk speed.

In another preferred embodiment of the invention and or embodiments thereof the microspheres
a) are prepared by spray drying and sieving.

For this, it is preferred that the physiologically active macrocyclic lactone (a1), polymer (a2) and optionally further physiologically acceptable excipient can be dissolved in a solvent, preferably acetone, and atomized with a conventional laboratory spray dryer spinning with the standard setups known to the skilled person. It is preferred that the above atomizing is conducted at a temperature of about 15°C to about 25°C, preferably at about 20°C.

### Aqueous carrier

In step iii) an aqueous carrier is prepared by dissolving one or more suspending agents and/or one or more wetting agents and optionally other excipients in water. It is preferred that one or more suspending agents and/or one or more wetting agents and optionally further physiologically acceptable excipient(s) are suspended in water or in water and a water-miscible liquid, preferably in water. As far as the water-miscible liquid is concerned, the same as described above applies. It is preferred that the above dissolving in step iii) is conducted at a temperature of about 20°C to about 30°C, preferably at about 23°C to obtain a mixture. It preferred that the above dissolving in step iii) is carried out under mechanical treatment such as stirring.

_In step iii) the particles obtained from step i) and the microspheres obtained from step ii) are mixed with the aqueous carrier obtained in step iii). The particles and microspheres can be added to the aqueous carrier or vice versa. Preferably the particles obtained from step i) and the microspheres obtained from step ii) are added to the aqueous carrier. It is preferred that the above adding in step iv) is conducted at a temperature of about 20°C to about 30°C, preferably at about 23°C to obtain a mixture. It is preferred that the above adding in step iv) is carried out under mechanical treatment such as stirring.

Another aspect of the invention is a kit comprising:
(A) a first container comprising
   - a mixture of microspheres as described in any one of claims 1 to 14 and particles of an isoxazoline compound of Formula (I) as described in any one of claim 1 to 14 and/or a compound of Formula (II) as described in any one of claims 1 to 14,
(B) a second container with an aqueous carrier comprising one or more wetting agents and/or one or more suspending agents and water and
(C) instructions for reconstituting the microspheres as described in any one of claims 1 to 14 and of particles of an isoxazoline compound of Formula (I) as described in any one of claims 1 to 14 or a compound of Formula (II) as described in any one of claims 1 to 14 with the aqueous carrier prior an injection.

As far as the components such as for example the physiologically active macrocyclic lactone (a1), the isoxazoline compound of Formula (I) and/or the suspending agent, their content and properties are concerned, the same as described above applies.

In one embodiment of the invention and/or embodiments thereof the kit further comprises an apparatus for reconstituting and parenterally administering a mixture of the composition from the first and second container to an animal, especially a syringe.

### Method of treatment

Another aspect of the present invention is the injectable veterinary composition of the present invention for use in treating and/or preventing a parasite infestation in an animal. The composition of this invention is administered parenterally via an injection.

A parasite infestation refers to the presence of parasites in numbers that pose a risk to humans or animals.

It has been shown in the Example that the composition of the invention results in effective blood plasma concentration of moxidectin and fluralaner for an extended period, for more than 6 months. This means the present injectable composition achieves an effective extended release effect of a physiologically active macrocyclic lactone (a1) and the isoxazoline compound according to Formula (I) or the compound according to Formula (II) against ectoparasites (such as fleas, ticks and mites) and endoparasites, such as worms (helminths).

The invention also provides a method for introducing and maintaining blood levels of a physiologically active macrocyclic lactone (a1) and an isoxazoline compound according to Formula (I), especially fluralaner and/or the compound according to Formula (II), especially of moxidectin and fluralaner, preferably (S)-fluralaner in animals for an extended period of time and a method for the prevention or treatment of infections and infestations caused by helminths, nematodes, and ectoparasitic arthropods in animals.

Additionally, it turned out that after injection the present injectable composition safe to the animal, shows a desirable bioavailability and duration of efficacy and does not cause inacceptable side effects, especially no inacceptable injection site irritation, wherein the present composition can be administered by subcutaneous or intramuscular injection. In one embodiment the suspension is administered by subcutaneous or intramuscular injection to an animal.

In addition, it has been discovered that a single administration of such compositions generally provides potent activity against one or more parasites (e.g. ectoparasites such as fleas, ticks or mites), while also tending to provide fast onset of activity, long duration of activity, and/or desirable safety profiles.

Finally, the new injectable composition allows the use of these modern compounds under conditions, where separate injections and a repeated administration is not desirable.

The present invention provides an injectable composition showing at least one of the above-mentioned advantageous properties.

Another aspect of the invention is the injectable veterinary composition of the present invention for use in treating and/or preventing a parasite infestation in an animal.

As far as the injectable veterinary composition is concerned, the same applies as described above. The same applies to parasites and parasite infestation. The term "treatment" as used herein refers to reversing, alleviating, inhibiting the parasite infestation. Prevention/ protection is stopping a new or incoming infestation or infection from establishing.

For an *in vivo* administration of the composition according to the invention, an effective amount is synonymous with a "therapeutically or prophylactically effective amount", which is the dose or amount that prevents or treats / ameliorates symptoms and/or signs of parasite infection or infestation by the treated animal or prevents a parasite infestation or reduces parasite numbers in and/or on an animal and/or to inhibits the development of parasite infestation in or on an animal, in whole or in part at a reasonable benefit/risk ratio applicable to any medical treatment.

An endoparasite which seriously harms animals is *Dirofilaria immitis,* also known as Heartworm. The most common hosts are dogs and cats but other animals such as ferrets and raccoons may also be infected. The parasitic worm is transmitted by the mosquito bites, which carry the heartworm larvae. The adult worms live in the major blood vessels of the lung, causing inflammation of the blood vessels and potentially resulting in heart damage and early death. In advanced infections, the worms enter the heart as well.

In a particularly preferred embodiment of the invention, the compositions of the invention are used to treat or prevent an infection by *Dirofilaria immitis.* In another embodiment the compounds and compositions of the invention are used to treat or prevent an infection by *Dirofilaria repens.*

Macrocyclic lactones are especially useful to control heartworm (*Dirofilaria* spp.) infestations in animals, especially pets or companion animals, especially in dogs or cats.

The concentration of the active ingredients in the composition needs to be sufficient to provide the desired therapeutically or prophylactically effective amount in a volume that is acceptable for injectable administration depending on the animal treated.

The injectable veterinary composition is administered parenterally, in particular by subcutaneous or intramuscular injection to the animal. A subcutaneous or intramuscular injectable administration can for example be carried out by a syringe.

The compositions according to the invention have good syringeability. The term "syringeable" describes a suspension that can be easily drawn from an ampoule/vial/container with a needle into a syringe and subsequently injected from such a syringe through the needle (e.g. 18 Gauge needle) intramuscularly (im) or subcutaneously (sc).

In a preferred embodiment of the invention and/or embodiments thereof, the dosage regime of the injectable veterinary composition is, monthly, semiannually or annually. Preferably the injectable pharmaceutical compositions may be administered every month, every two months, every three months, every four months, every five months, every six months, every seven months, eight months, every nine months, every ten months, every eleven months, every twelve months, every 13 months, every 14 months, every 15 months, every 16 months, every 17 months or every 18 months, in particular once every six months or once every twelve months.

Especially preferred is an administration every 6 months. Preferred is also an administration or every 12 months. In one embodiment the dosage regime is at least once every six months or once every twelve months.

This provides a long- term protection of animals from both ectoparasites, especially fleas and ticks, and endoparasites, especially heartworm and/or gastrointestinal helminths. Especially preferred is long term protection against heartworm infestation.

Of benefit is the possibility to apply the injectable composition of the invention together with the annual vaccination against infectious diseases such as distemper, influenza, rabies and other vaccines with conventional antigens.

In a preferred embodiment of the invention and/or embodiments thereof, the animal is a pet. A pet (also referred to as companion animal) can be regarded as an animal generally living in or close to the habitation of its owner. Examples of pets include, but are not limited to, dogs, cats, rabbits, guinea pigs and birds such as budgies and parrots.

In one embodiment the animal is a pet. In one embodiment the animal is a dog or cat. In a more preferred embodiment of the invention and/or embodiments thereof, the animal is a dog.

In a preferred embodiment of the invention and/or embodiments thereof, the isoxazoline compound of Formula (I), preferably fluralaner, and/or the compound according to Formula (II) is administered at about 0.01 to about 200 mg/kg body weight of the animal, preferably from about 0.1 to about 100 mg per kg of animal body weight, more preferably from about 0.5 to about 50 mg per kg of animal body weight, in particular from about 1 to about 30 mg per kg of animal body weight. The total dose can be given at once or in divided doses.

In a preferred embodiment of the invention and/or embodiments thereof, the physiologically active macrocyclic lactone, preferably moxidectin is administered at about 0.01 to about 10 mg/kg body weight of the animal, preferably from about 0.1 to about 5 mg per kg of animal body weight.

It turned out that when the present injectable veterinary composition is used in treating and/or preventing a parasite infestation in an animal, the treated animal suffers minimal injection site irritation.

Injection site irritation is an injury produced at the injection site and surrounding tissue, when an animal receives an injection of a pharmaceutical composition. Such injury can be swelling, skin discoloration and tissue necrosis. Though some injection site irritation is inevitable in some animals, injection site swelling of more than 2 x 2 cm that further persists for more than two to three days is generally considered to be unacceptable by veterinarians and animal owners.

Another aspect of the present invention is a method for treating and/or preventing a parasite infestation in an animal comprising administering to a subject in need thereof a therapeutically effective amount of the injectable veterinary composition according to the present invention or the kit according to the present invention

Again, as far as the injectable veterinary composition and the kit are concerned, the same applies as described above. The same applies to parasites and parasite infestation.

Thus, the invention provides a method of treating and/or preventing a parasite infestation which comprises administering to an animal a therapeutically effective amount of the injectable veterinary composition of the present invention or using the kit according to the present invention.

Features of the invention have been described in embodiments in the present application; however, for brevity not all combinations of the features are literally described. Combinations of features as described above are, however, expressly considered to be part of the invention.

### EXAMPLES

### Example 1: Manufacture of microspheres comprising moxidectin and polycaprolactone (PCL) by solvent evaporation

11.25 g of polyvinyl alcohol were dissolved in 1500 mL of deionized water. Separately, 4.5 g of polycaprolactone and 0.5 g of moxidectin were dissolved in 50 mL of dichloromethane. With overhead stirring of the aqueous solution at 330 rpm and at 20°C, the solution comprising dichloromethane was added dropwise to the aqueous solution. The resulting emulsion was stirred for four hours. The microspheres were isolated over a 75 µm sieve and rinsed with deionized water followed by air drying at room temperature.

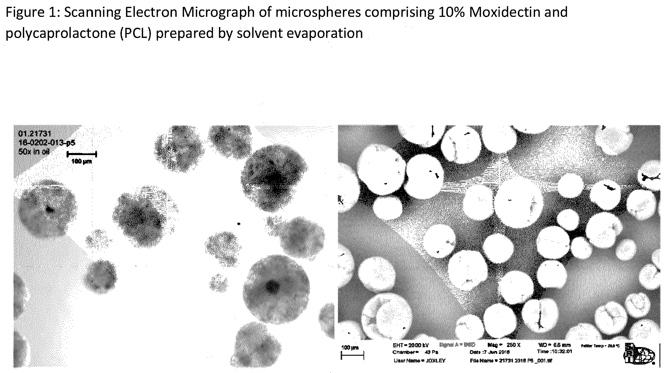

The SEM image of the resulting microspheres is shown in Figure 1.

The particle size analysis showed the following values: D10-value: 117.66 µm; D50-value: 190.48 µm; D90-value: 273.43 µm.

### Example 2: Manufacture of microspheres comprising moxidectin and polycaprolactone (PCL) by spinning disk atomization using dichloromethane

6.0 g of moxidectin and 54.0 g of polycaprolactone were dissolved in 690 mL of dichloromethane and cooled to 4°C. The solution was fed at 150 g/min onto a four-inch diameter custom disc spinning at 2500 rpm from a height of 30 ft. The atomization space was cooled to 19°C. The resulting particles were collected and passed through a 420 µm sieve and then a 250 µm sieve to obtain the microspheres.

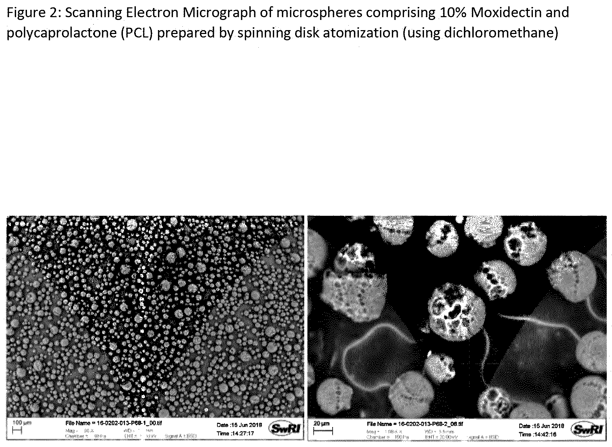

The SEM image of the resulting microspheres is shown in Figure 2.

The particle size analysis showed the following values: D10-value: 41.19 µm; D50-value: 68.01 µm; D90-value: 113.89 µm.

### Example 3: Manufacture of microspheres comprising moxidectin and polycaprolactone (PCL) by spinning disk atomization using acetone

2.5 g of moxidectin and 22.5 g of polycaprolactone were dissolved in 475 mL of acetone and cooled to 4°C. A custom stainless- steel spinning disc atomization chamber (approximately 4 ft diameter cone) was heated to 35°C, housing and spinning disc at the top center and cyclone collector connected to the bottom of the cone. The solution was pumped at 50 g/min onto a three-inch diameter stainless steel disc spinning at approximately 5000 rpm. The resulting particles were collected to obtain the microspheres.

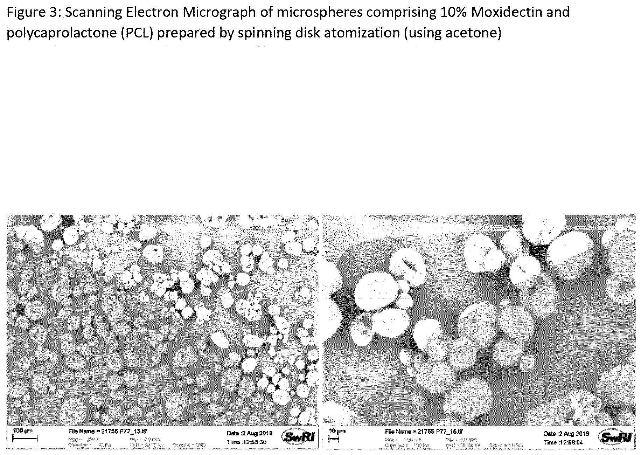

The SEM image of the resulting microspheres is shown in Figure 3.

The particle size analysis showed the following values: D10-value: 31.23 µm; D50-value: 58.65 µm; D90-value: 105.44 µm.

### EXAMPLE 4: Manufacture of microspheres comprising moxidectin and polycaprolactone (PCL) by spray drying

37.0 g of polycaprolactone and 4.2 g of moxidectin were dissolved in 800 mL of acetone. The mixture was atomized at 6-7 g/min through a 600 µm bifluid nozzle (3.2 bar nozzle pressure) into a Pro-C-epT 4M8 laboratory spray drier with an inlet temperature of 25°C, chamber temperature of 19°C and an outlet temperature of 19.54°C. The resulting particles were collected to obtain the microspheres.

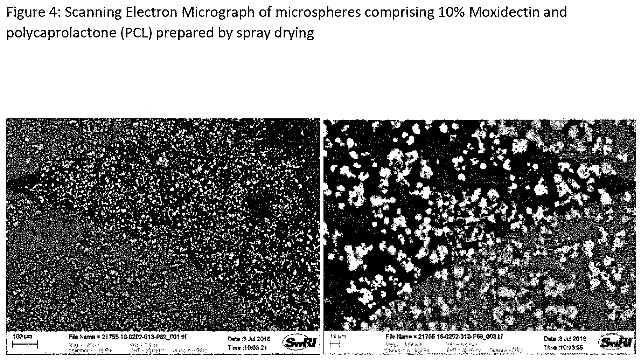

The SEM image of the resulting microspheres is shown in Figure 4.

The particle size analysis showed the following values: D10-value: 4.54 µm; D50-value: 11.16 µm; D90-value: 33.94 µm.

### EXAMPLE 5: Sterilization of microspheres comprising moxidectin and polycaprolactone (PCL)

Samples of microspheres comprising moxidectin and polycaprolactone (PCL) and samples pf moxidectin drug substance were placed in 20 mL serum vials. Then, the microspheres comprising moxidectin and polycaprolactone were irradiated 15, 20, and 25 kGy irradiation by both gamma irradiation and electron-beam for sterilization. The microspheres were sterilized either at 5°C or ambient temperature (23°C) and with or without nitrogen overlay. Samples were assessed for changes in assay. % assay reported as % of non-irradiated assay. The results are shown in below Table 1

CONCLUSION: Moxidectin PCL microspheres can be sterilized using both irradiation methods. The moxidectin loss was higher in the microspheres sterilized with gamma irradiation than those exposed to E-beam. E-beam irradiation involved the use of higher dose rate causing less exposure time and reducing the potential degradation of the drug. It was also observed that degradation was dependent from the irradiation dose. Sparging the microspheres with nitrogen did not did not show an effect on stability.

**Table 1: Assay results of the samples subjected to electron-beam and gamma irradiation**

| **Electron Beam** | | | | | | **Gamma Irradiation** | | | | |
|---|---|---|---|---|---|---|---|---|---|---|
| **Microsphere Lot** | **Dose (kGγ)** | **Temperature** | **Sparge** | **Assay** | | **Microsphere Lot** | **Dose (kGγ)** | **Temperature** | **Sparge** | **Assay** |
| 10% Moxidectin in PCL Microspheres | 15 | amb | not sparged | 95.39 | | 10% Moxidect in PCL Microspheres | 15-18 | amb | not sparged | 69.74 |
| | | | nitrogen overlay | 96.80 | | | | | nitrogen overlay | 70.30 |
| | | 5°C | not sparged | 92.33 | | | | 5°C | not sparged | 73.57 |
| | | | nitrogen overlay | 91.59 | | | | | nitrogen overlay | 74.62 |
| | 20 | amb | not sparged | 90.09 | | | 20-24 | amb | not sparged | 51.64 |
| | | | nitrogen overlay | 88.97 | | | | | nitrogen overlay | 51.86 |
| | | 5°C | not sparged | 89.30 | | | | 5°C | not sparged | 56.24 |
| | | | nitrogen overlay | 90.99 | | | | | nitrogen overlay | 57.59 |
| | 25 | amb | not sparged | 87.85 | | | 25-30 | amb | not sparged | 45.97 |
| | | | nitrogen overlay | 88.04 | | | | | nitrogen overlay | 51.59 |
| | | 5°C | not sparged | 87.61 | | | | 5°C | not sparged | 49.48 |
| | | | nitrogen overlay | 88.22 | | | | | nitrogen overlay | 58.84 |
| | 15 | amb | not sparged | 96.08 | | | 15-18 | amb | not sparged | 97.18 |
| | | | nitrogen overlay | 94.98 | | | | | nitrogen overlay | 97.69 |
| | | 5°C | not sparged | 93.42 | | | | 5°C | not sparged | 95.59 |
| | | | nitrogen overlay | 93.06 | | | | | nitrogen overlay | 95.76 |
| | 20 | amb | not sparged | 95.31 | | | 20-24 | amb | not sparged | 95.51 |
| Moxidectin Drug Substance | | | nitrogen overlay | 95.91 | | Moxidectin Drug Substance | | | nitrogen overlay | 96.67 |
| | | 5°C | not sparged | 94.07 | | | | 5°C | not sparged | 94.30 |
| | | | nitrogen overlay | 94.03 | | | | | nitrogen overlay | 93.64 |
| | 25 | amb | not sparged | 94.94 | | | 25-30 | amb | not sparged | 95.23 |
| | | | nitrogen overlay | 95.11 | | | | | nitrogen overlay | 96.03 |
| | | 5°C | not sparged | 93.15 | | | | 5°C | not sparged | 93.13 |
| | | | nitrogen overlay | 93.48 | | | | | nitrogen overlay | 95.06 |

### EXAMPLE 6: Preparation of liquid aqueous carrier (vehicle)

Example formulations of aqueous carriers (vehicle for reconstitution/resuspension):

| Ingredients | % w/w |
|---|---|
| Na CMC | 2.2 |
| Poloxamer 124 | 0.11 |
| Sodium phosphate (dibasic dihydrate) | 0.77 |
| Benzyl alcohol | 2.2 |
| HCl | 0.17 |
| WFI | QS |

Approximately 50% of the water for injection was charged to a vessel and heated to about 70-80°C, and the suspending agent sodium carboxymethyl cellulose (NaCMC), Poloxamer 124 was added and homogenized until dissolved. The other ingredients were added slowly and mixed with stirring to achieve dispersion. The heat was removed and cold water for injection added to bring the volume to 10 liters. The pH was adjusted to 4.5-5.5 by adding HCl.

The vehicle was the sterilized by autoclave and the vehicle solution stored in sterile containers.

### Example 7: Preparation of particles of fluralaner

Fluralaner was dissolved at 73 - 77°C in iso-propanol. The solution was filtered and transferred into the crystallizer reactor. The mixture was cooled down to 48-52 °C and seeded. After 30 min post-seeding aging, the mixture was cooled down to 20°C (seed bed). One portion of the resulting suspension was transferred into a second reactor (dissolver) and heated up to 70°C, until complete dissolution was achieved. The remaining slurry into the crystallizer was heated up to 54°C. The crystallizer content was transferred to the dissolver and back to the crystallizer, with a recirculation constant flow rate of 0.40 BV/h (batch volume = 10 V → 4.0 V/h), for 2.5 h. Once completed the transfer of the solution from dissolver to crystallizer, the slurry was aged at 54°C for 5 h. Then, a very slow cooling ramp was started: from 54°c to 45°C over 6 h (0.025°C/min) and from 45°C to 0 °C over 15 h (3 °C/hr). The slurry was aged at -10°C for 1 h, then the product was isolated through filtration, without applying washing. The wet product was then dried.

### Example 8: Preparation of Fluralaner particles in PLGA Microspheres

16.0 g of fluralaner and 16.0 g of poly(lactide-co-glycolide) 50:50 were dissolved into 285.0 g of dichloromethane. The solution was fed at approximately 100 g/min onto a 4 inch diameter custom disc spinning at approximately 3000 rpm. The disc was mounted in a custom polyethylene square (4x4x4 ft.) enclosure with a 60° angled bottom cone. The enclosure was warmed with dry air to 54.2-54.7 °C at the top and 36.3-38.1 °C at the bottom of the enclosure during the course of the atomization.

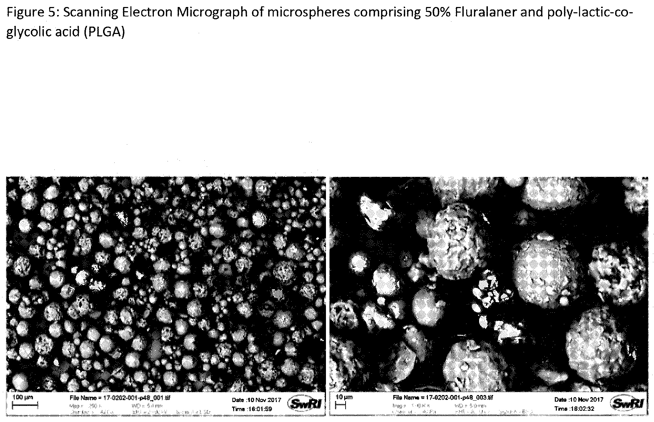

The SEM image of the resulting microspheres is shown in Figure 5.

### Example 9: Preparation an injectable composition according to the invention and Determination of Dose accuracy

The vial comprising moxidectin microspheres from Example 3 and fluralaner particles from Example 7 were reconstituted with 17 mL of the aqueous carrier of Example 6. During reconstitution the vial containing the moxidectin microspheres and fluralaner particles was rotated while being held horizontally throughout carrier addition.

Once all the carrier was added, the vial was shaken for 1 minute.

A dose accuracy study was conducted to demonstrate that the correct amount of moxidectin and fluralaner was dispensed when dosing 1 mL of reconstituted composition comprising moxidectin and fluralaner.

Herein, the vial with the reconstituted composition as described above was manipulated in one of the following ways:
- vigorously hand shaken for 1, 2 or 3 minutes prior to sampling;
- vigorously hand shaken for 3 minutes and mixed using the syringe 5 times prior to sampling; or
- vigorously hand shaken for 3 minutes, mixed using the syringe 5 times and vortexed for 20 seconds prior to sampling.

After the solid material was completely suspended, six 1 mL doses were sampled from the vial. Prior to each dose, the vial was vigorously shaken for 15 seconds to avoid settling between samples.

Each of the six doses were transferred to a separate 50 mL volumetric flask along with 10 mL of water: then the samples were diluted to volume with 50/50 acetonitrile/isopropanol and sonicated for 20 minutes.

These samples were analysed to determine the moxidectin concentration.

Additionally, 1 mL of the samples were transferred into separate 25 mL volumetric flasks and diluted with 40/40/20 acetonitrile/isopropanol/water. The fluralaner concentrations were determined from these diluted samples.

Table 2 below presents the moxidectin and fluralaner concentration results and shows that all manipulation of the samples resulted in a uniform active ingredient concentration in the sample aliquots, hence the injectable composition is easily re-suspendable and uniform.

**Table 2: Moxidectin and fluralaner concentration results**

| **Reconstitution Procedure** | **Dose #** | **Moxidectin Percent Label Claim** | **Fluralaner Percent Label Claim** |
|---|---|---|---|
| hand shaken for 1 minute | 1 | 103.25 | 102.68 |
| | 2 | 101.25 | 101.83 |
| | 3 | 103.84 | 106.97 |
| | 4 | 101.70 | 103.52 |
| | 5 | 103.45 | 104.38 |
| | 6 | 89.32 | 90.80 |
| hand shaken for 2 minutes | 1 | 101.80 | 80.44 |
| | 2 | 102.26 | 103.67 |
| | 3 | 100.54 | 102.82 |
| | 4 | 101.89 | 84.95 |
| | 5 | 96.62 | 99.01 |
| | 6 | 104.02 | 107.40 |
| | 1 | 99.92 | 95.36 |
| hand shaken for 3 minutes | 2 | 98.29 | 102.59 |
| | 3 | 98.52 | 95.41 |
| | 4 | 98.71 | 99.43 |
| | 5 | 101.21 | 108.06 |
| | 6 | 85.54 | 88.68 |
| hand shaken for 3 minutes and mixed using a syringe | 1 | 98.43 | 105.95 |
| | 2 | 98.31 | 104.31 |
| | 3 | 95.62 | 89.74 |
| | 4 | 98.17 | 105.87 |
| | 5 | 97.67 | 104.26 |
| | 6 | c95.90c | 103.83 |
| hand shaken for 3 minutes, mixed using a syringe, and vortexed | 1 | 93.87 | 100.86 |
| | 2 | 97.78 | 106.80 |
| | 3 | 97.72 | 105.14 |
| | 4 | 99.89 | 105.86 |
| | 5 | 98.19 | 106.37 |
| | 6 | 95.12 | 104.61 |

### Example 10: Pharmacokinetic Assessment of microspheres comprising moxidectin microspheres in polycaprolactone and microspheres comprising fluralaner and poly (lactic-co-glycolic acid)

### Example 10.1:

A ready-to-use injectable suspension of moxidectin-comprising microspheres manufactured as in Example 1 and fluralaner in PLGA microspheres manufactured as shown in Example 8 was administered subcutaneously on a single occasion to three Beagle dogs at 10 mg/kg body weight (BW) of fluralaner and 0.17 mg/kg BW of moxidectin. The local tolerance of the test article was assessed at intervals up to 84 days.

Blood samples for determination of fluralaner and moxidectin plasma concentrations were collected on study days 1, 3, 4, 5, 7, 10, 14, 21, 28, 35, 43, 49, 56, 70, 84, 98, 112, 126, 140, 154, 168 and 182.

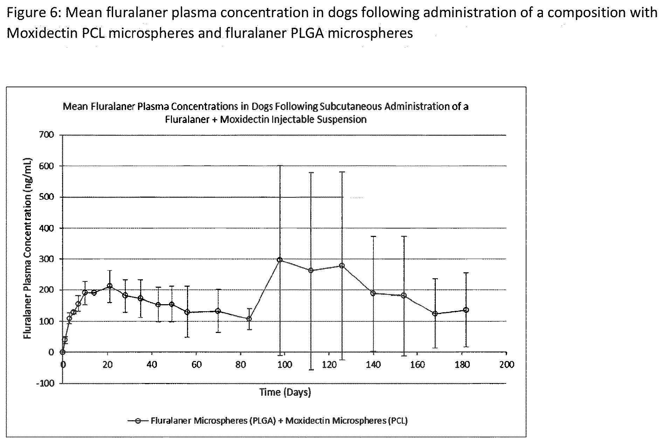

The fluralaner and moxidectin plasma concentrations are shown in Figures 5 and 6, respectively.

A favorable pharmacokinetic profile showing prolonged moxidectin plasma concentration was obtained after subcutaneous administration of the 10% Moxidectin in PCL microspheres prepared by solvent evaporation.

### Example 10.2:

Three injectable suspensions of moxidectin-comprising microspheres manufactured as in Example 2 and 3 and fluralaner particles manufactured as in Example 7 were reconstituted with 17 mL of the aqueous carrier of Example 6 and administered subcutaneously on a single occasion to eight Beagle dogs at 15 mg/kg BW of fluralaner and 0.17 mg/kg BW of moxidectin.

Group 1: Microspheres correspond to example 3.

Group 2 and 3 - the microspheres correspond to example 2 and in this case group 2 was irradiated at 15kG and group 3 was irradiated at 25kG.

The local tolerance of the test articles was assessed through 54 days.

Blood samples for determination of fluralaner and moxidectin plasma concentrations were collected on study days 1 (pre dose and 8 hours post dose), 2, 4, 6, 8, 11, 15, 22, 29, 36, 43, 50, 57, 71, 85, 99, 113, 127, 141, 155, 169 and 183.

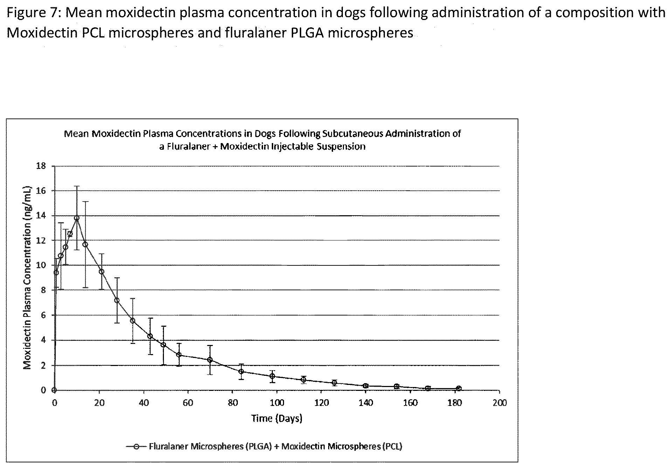

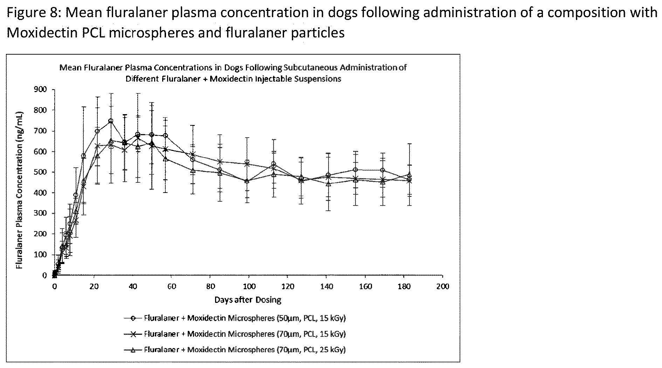

The fluralaner and moxidectin plasma concentrations are shown in Figures 7 and 8, respectively.

Moxidectin concentration declined at faster rate from the moxidectin in PCL microspheres prepared by spinning disk atomization as compared to solvent evaporation.

However, favorable pharmacokinetic profiles showing prolonged plasma concentrations of fluralaner were obtained for all formulations.

CONCLUSION: The combination of 10% Moxidectin in PCL microspheres prepared by solvent evaporation with fluralaner particles provides prolonged plasma concentrations in canine for up to six months.

## Claims

1. An injectable veterinary pharmaceutical composition comprising
(a) microspheres comprising
(a1) about 1% to about 40% w/w, based on the weight of the microspheres, of one or more physiologically active macrocyclic lactone(s), and
(a2) about 60% to about 99% w/w, based on the weight of the microspheres, of polycaprolactone (PCL),
and
(b) particles of an isoxazoline compound of Formula (I) 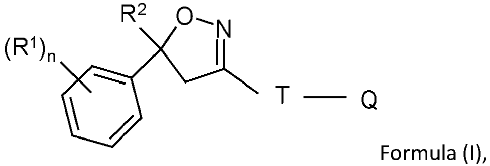 wherein
R¹ = halogen, CF₃, OCF₃, CN,
n = integer from 0 to 3, preferably 1, 2 or 3,
R² = C₁-C₃-haloalkyl, preferably CF₃ or CF₂Cl,
T = 5 to 12-membered mono or bicyclic ring system which is optionally substituted by one or more radicals Y,
Y = methyl, halomethyl, halogen, CN, NO₂, NH₂-C=S, or two adjacent radicals Y form together a chain, especially a three or four-membered chain;
Q = X-NR³R⁴ ora 5-membered N-heteroaryl ring which is optionally substituted by one or more radicals;
X = CH₂, CH(CH₃), CH(CN), CO, CS,
R³ = hydrogen, methyl, haloethyl, halopropyl, halobutyl, methoxymethyl, methoxyethyl, halomethoxymethyl, ethoxymethyl, haloethoxymethyl, propoxymethyl, ethylaminocarbonylmethyl, ethylaminocarbonylethyl, dimethoxyethyl, propynylaminocarbonylmethyl, N-phenyl-N-methyl-amino, haloethylaminocarbonylmethyl, haloethylaminocarbonylethyl, tetrahydrofuryl, methylaminocarbonylmethyl, (N,N-dimethylamino)-carbonylmethyl, propylaminocarbonylmethyl, cyclopropylaminocarbonylmethyl, propenylaminocarbonylmethyl, haloethylaminocarbonylcyclopropyl, 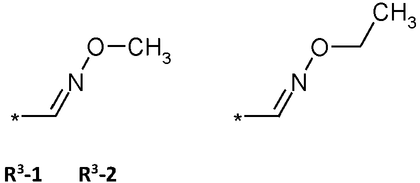 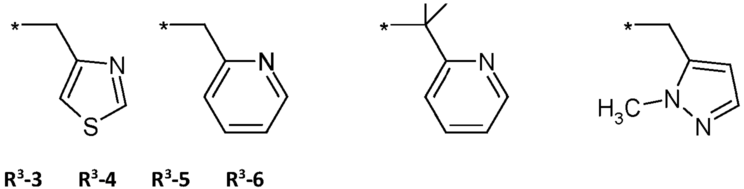 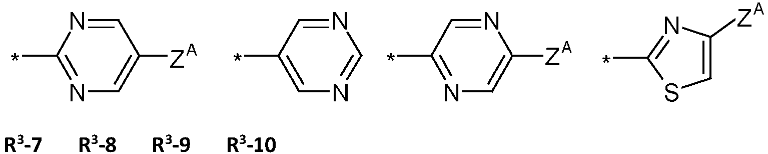 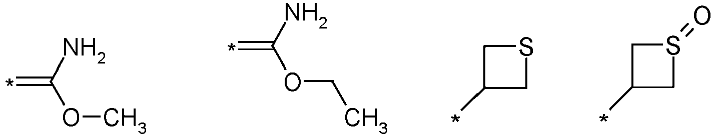 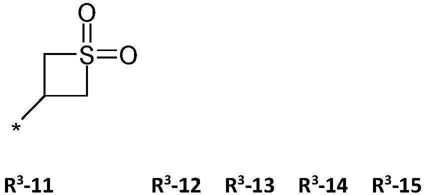
wherein Z^{A} = hydrogen, halogen, cyano, halomethyl (CF₃);
R⁴ = hydrogen, ethyl, methoxymethyl, halomethoxymethyl, ethoxymethyl, haloethoxymethyl, propoxymethyl, methylcarbonyl, ethylcarbonyl, propylcarbonyl, cyclopropylcarbonyl, methoxycarbonyl, methoxymethylcarbonyl, aminocarbonyl, ethylaminocarbonylmethyl, ethylaminocarbonylethyl, dimethoxyethyl, propynylaminocarbonylmethyl, haloethylaminocarbonylmethyl, cyanomethylaminocarbonylmethyl, or haloethylaminocarbonylethyl; or
R³ and R⁴ together form a substituent selected from the group consisting of: 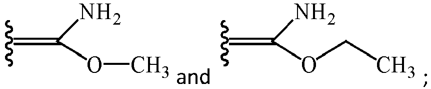 and
c) an aqueous carrier;
wherein the microspheres (a) and the particles of compound (b) are suspended in the aqueous carrier (c).

2. The injectable veterinary composition according to claim 1, wherein the macrocyclic lactone is moxidectin.

3. The injectable veterinary composition according to any one of claims 1 to 2, wherein the D50 of the volume weighted particle size distribution of the microspheres (a) is from about 8 µm to about 250 µm, more preferably from about 20 µm to about 200 µm, even more preferably from about 40 µm to about 80 µm, in particular from about 50 µm to about 70 µm.

4. The injectable veterinary composition according to any one of claims 1 to 3, wherein the isoxazoline compound according to Formula (I) is selected from the group consisting of fluralaner, afoxolaner, sarolaner, and lotilaner.

5. The injectable veterinary composition according to any one of claims 1 to 4, wherein the aqueous carrier comprises a suspending agent and the suspending agent is selected from sodium carboxymethylcellulose, polyvinylpyrrolidone, methylcellulose and mixtures thereof.

6. The injectable veterinary composition according to any one of claims 1 to 5, wherein the aqueous carrier comprises a wetting agent and the wetting agent is a poloxamer.

7. A method of preparing the injectable veterinary composition according to any one of claims 1 to 6 comprising the steps of:
i) preparing microspheres (a) of a macrocyclic lactone by solvent evaporation, spinning disk atomization or spray drying, and optionally sieving the resulting product,
ii) preparing particles (b) of the isoxazoline compound of Formula (I),
iii) preparing the aqueous carrier by dissolving one or more suspending agents and/or one or more wetting agents in water, and
iv) mixing the particles obtained from step i) and the microspheres obtained from step ii) with the aqueous carrier obtained in step iii).

8. The method according to claim 7 wherein the macrocyclic lactone is moxidectin and the isoxazoline compound according to Formula (I) is selected from the group consisting of fluralaner, afoxolaner, sarolaner, and lotilaner.

9. The method according to claim 7 or 8, wherein the wetting agent is a poloxamer and the suspending agent is selected from sodium carboxymethylcellulose, polyvinylpyrrolidone, methylcellulose and mixtures thereof

10. A kit, wherein the kit comprises:
(A) a first container comprising a mixture of microspheres and particles of an isoxazoline compound of Formula (I) as defined in any one of claim 1 to 6,
(B) a second container with an aqueous carrier as defined in any one of claim 1 to 6; and
(C) instructions for reconstituting the microspheres and the particles in the aqueous carrier prior to an injection to an animal.

11. The injectable veterinary composition of claims 1 to 6 for use in treating and/or preventing a parasite infestation in an animal.

12. The injectable veterinary composition for use according to claim 11, wherein the injectable veterinary composition is administered by a subcutaneous or intramuscular injection to the animal.

13. The injectable veterinary composition for use according to claim 11 or 12, wherein the dosage regime of the injectable veterinary composition is once every six months or once every twelve months.

14. The injectable veterinary composition for use according to any one of claims 11 to 13, wherein the animal is a pet.

15. The injectable veterinary composition for use according to claim 14, wherein the animal is a dog.

## Patentansprüche

1. Injizierbare veterinärmedizinische pharmazeutische Zusammensetzung, umfassend
(a) Mikrokügelchen, umfassend
(al) etwa 1 Gew.-% bis etwa 40 Gew.-%, bezogen auf das Gewicht der Mikrokügelchen, eines oder mehrerer physiologisch wirksamer makrocyclischer Lactone und
(a2) etwa 60 Gew.-% bis etwa 99 Gew.-%, bezogen auf das Gewicht der Mikrokügelchen, Polycaprolacton (PCL),
und
(b) Partikel einer Isoxazolinverbindung der Formel (I) 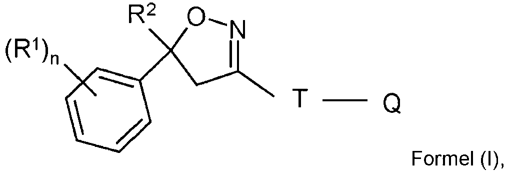 wobei
R¹ = Halogen, CF₃, OCF₃, CN,
n = eine ganze Zahl von 0 bis 3, vorzugsweise 1, 2 oder 3,
R² = C₁-C₃-Halogenalkyl, vorzugsweise CF₃ oder CF₂Cl,
T = ein 5- bis 12-gliedriges mono- oder bicyclisches Ringsystem, das gegebenenfalls durch einen oder mehrere Reste Y substituiert ist,
Y = Methyl, Halogenmethyl, Halogen, CN, NO₂, NH₂-C=S, oder zwei benachbarte Reste Y bilden zusammen eine Kette, insbesondere eine drei- oder viergliedrige Kette,
Q = X-NR³R⁴ oder ein 5-gliedriger N-Heteroarylring, der gegebenenfalls durch einen oder mehrere Reste substituiert ist,
X = CH₂, CH(CH₃), CH(CN), CO, CS,
R³ = Wasserstoff, Methyl, Halogenethyl, Halogenpropyl, Halogenbutyl, Methoxymethyl, Methoxyethyl, Halogenmethoxymethyl, Ethoxymethyl, Halogenethoxymethyl, Propoxymethyl, Ethylaminocarbonylmethyl, Ethylaminocarbonylethyl, Dimethoxyethyl, Propinylaminocarbonylmethyl, N-Phenyl-N-methylamino, Halogenethylaminocarbonylmethyl, Halogenethylaminocarbonylethyl, Tetrahydrofuryl, Methylaminocarbonylmethyl, (N,N-Dimethylamino)carbonylmethyl, Propylaminocarbonylmethyl, Cyclopropylamino-carbonylmethyl, Propenylaminocarbonylmethyl, Halogenethylaminocarbonylcyclopropyl, 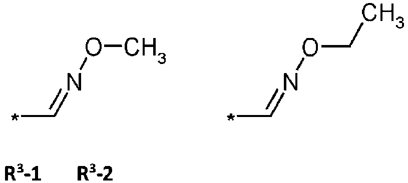 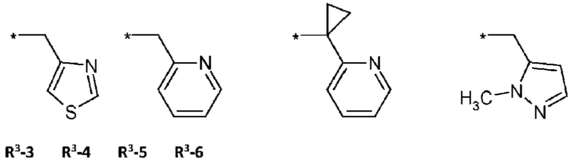 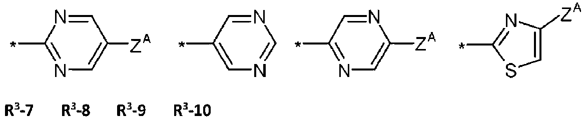 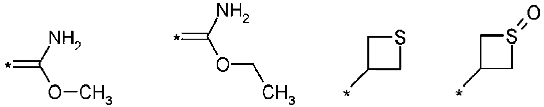 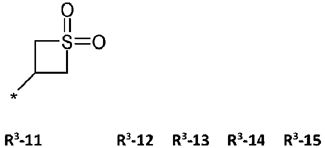
wobei Z^{A} = Wasserstoff, Halogen, Cyano, Halogenmethyl (CF₃),
R⁴ = Wasserstoff, Ethyl, Methoxymethyl, Halogenmethoxymethyl, Ethoxymethyl, Halogenethoxymethyl, Propoxymethyl, Methylcarbonyl, Ethylcarbonyl, Propylcarbonyl, Cyclopropylcarbonyl, Methoxycarbonyl, Methoxymethylcarbonyl, Aminocarbonyl, Ethylaminocarbonylmethyl, Ethylaminocarbonylethyl, Dimethoxyethyl, Propinylaminocarbonylmethyl, Halogenethylaminocarbonylmethyl, Cyanomethylaminocarbonylmethyl oder Halogenethylaminocarbonylethyl oder
R³ und R⁴ zusammen einen aus der aus: 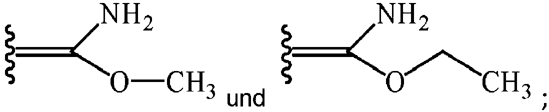 und
bestehenden Gruppe ausgewählten Substituenten bilden; und
c) einen wässrigen Träger;
wobei die Mikrokügelchwen (a) und die Partikel der Verbindung (b) in dem wässrigen Träger (c) subsendiert sind.

2. Injizierbare veterinärmedizinische Zusammensetzung nach Anspruch 1, wobei es sich bei dem makrocyclischen Lacton um Moxidectin handelt.

3. Injizierbare veterinärmedizinische Zusammensetzung nach Anspruch 1 oder 2, wobei der D50 der volumengewichteten Partikelgrößenverteilung der Mikrokügelchen (a) etwa 8 µm bis etwa 250 µm, besonders bevorzugt etwa 20 µm bis etwa 200 µm, noch mehr bevorzugt etwa 40 µm bis etwa 80 µm, insbesondere etwa 50 µm bis etwa 70 µm beträgt.

4. Injizierbare veterinärmedizinische Zusammensetzung nach einem der Ansprüche 1 bis 3, wobei die Isoxazolinverbindung der Formel (I) aus der aus Fluralaner, Afoxolaner, Sarolaner und Lotilaner bestehenden Gruppe ausgewählt ist.

5. Injizierbare veterinärmedizinische Zusammensetzung nach einem der Ansprüche 1 bis 4, wobei der wässrige Träger ein Suspendiermittel umfasst und das Suspendiermittel aus Natriumcarboxymethylcellulose, Polyvinylpyrrolidon, Methylcellulose und Mischungen davon ausgewählt ist.

6. Injizierbare veterinärmedizinische Zusammensetzung nach einem der Ansprüche 1 bis 5, wobei der wässrige Träger ein Netzmittel umfasst und wobei es sich bei dem Netzmittel um ein Poloxamer handelt.

7. Verfahren zur Herstellung der injizierbaren veterinärmedizinischen Zusammensetzung nach einem der Ansprüche 1 bis 6, welches die folgenden Schritte umfasst:
i) die Herstellung von Mikrokügelchen (a) eines makrocyclischen Lactons durch Lösungsmittelabdampfen, Drehscheibenzerstäubung oder Sprühtrocknung und gegebenenfalls Sieben des erhaltenen Produkts,
ii) die Herstellung von Partikeln (b) der Isoxazolinverbindung der Formel (I),
iii) die Herstellung des wässrigen Trägers durch Lösen von einem oder mehreren Suspendiermitteln und/oder einem oder mehreren Netzmitteln in Wasser und
iv) das Mischen der aus Schritt i) erhaltenen Partikel und der aus Schritt ii) erhaltenen Mikrokügelchen mit dem in Schritt iii) erhaltenen wässrigen Träger.

8. Verfahren nach Anspruch 7, wobei es sich bei dem makrocyclischen Lacton um Moxidectin handelt und die Isoxazolinverbindung der Formel (I) aus der aus Fluralaner, Afoxolaner, Sarolaner und Lotilaner bestehenden Gruppe ausgewählt ist.

9. Verfahren nach Anspruch 7 oder 8, wobei es sich bei dem Netzmittel um ein Poloxamer handelt und das Suspendiermittel aus Natriumcarboxymethylcellulose, Polyvinylpyrrolidon, Methylcellulose und Mischungen davon ausgewählt ist.

10. Kit, wobei das Kit Folgendes umfasst:
(A) einen ersten Behälter, umfassend eine Mischung von Mikrokügelchen und Partikeln einer wie in einem der Ansprüche 1 bis 6 definierten Isoxazolinverbindung der Formel (I),
(B) einen zweiten Behälter mit einem wie in einem der Ansprüche 1 bis 6 definierten wässrigen Träger und
(C) Anweisungen zum Rekonstituieren der Mikrokügelchen und der Partikel in dem wässrigen Träger vor einer Injektion in ein Tier.

11. Injizierbare veterinärmedizinische Zusammensetzung nach einem der Ansprüche 1 bis 6 zur Verwendung bei der Behandlung und/oder Prävention eines Parasitenbefalls in einem Tier.

12. Injizierbare veterinärmedizinische Zusammensetzung zur Verwendung nach Anspruch 11, wobei die injizierbare veterinärmedizinische Zusammensetzung durch subkutane oder intramuskuläre Injektion an ein Tier verabreicht wird.

13. Injizierbare veterinärmedizinische Zusammensetzung zur Verwendung nach Anspruch 11 oder 12, wobei das Verabreichungsschema der injizierbaren veterinärmedizinische Zusammensetzung einmal alle sechs Monate oder einmal alle zwölf Monate ist.

14. Injizierbare veterinärmedizinische Zusammensetzung zur Verwendung nach einem der Ansprüche 11 bis 13, wobei es sich bei dem Tier um ein Haustier handelt.

15. Injizierbare veterinärmedizinische Zusammensetzung zur Verwendung nach Anspruch 14, wobei es sich bei dem Tier um einen Hund handelt.

## Revendications

1. Composition pharmaceutique vétérinaire injectable comprenant
(a) des microsphères comprenant
(a1) environ 1 % à environ 40 % p/p, sur la base du poids des microsphères, d'une ou plusieurs lactones macrocycliques physiologiquement actives, et
(a2) environ 60 % à environ 99 % p/p, sur la base du poids des microsphères, de polycaprolactone (PCL),
et
(b) des particules d'un composé d'isoxazoline de formule (I) 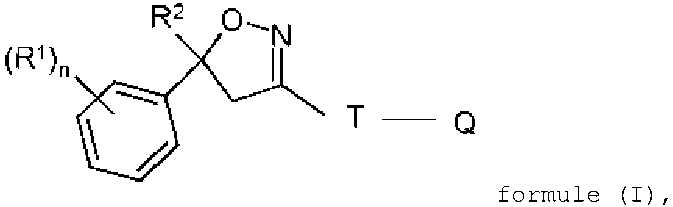
R1 = halogène, CF₃, OCF₃, CN,
n = entier de 0 à 3, préférablement 1, 2 ou 3, R2 = halogénoalkyle en C1-3, préférablement CF3 ou CF2Cl,
T = système cyclique monocyclique ou bicyclique à 5 à 12 chaînons qui est éventuellement substitué par un ou plusieurs radicaux Y,
Y = méthyle, halogénométhyle, halogène, CN, NO2, NH2-C=S, ou deux radicaux adjacents Y formant ensemble une chaîne, notamment une chaîne à trois ou quatre chaînons ;
Q = X-NR3R4 ou un cycle N-hétéroaryle à 5 chaînons qui est éventuellement substitué par un ou plusieurs radicaux ;
X = CH₂, CH(CH₃), CH(CN), CO, CS,
R3 = hydrogène, méthyle, halogénoéthyle, halogénopropyle, halogénobutyle, méthoxyméthyle, méthoxyéthyle, halogénométhoxyméthyle, éthoxyméthyle, halogénoéthoxyméthyle, propoxyméthyle, éthylaminocarbonylméthyle, éthylaminocarbonyléthyle, diméthoxyéthyle, propynylaminocarbonylméthyle, N-phényl-N-méthyl-amino, halogénoéthylaminocarbonylméthyle, halogénoéthylaminocarbonyléthyle, tétrahydrofuryle, méthylamino-carbonylméthyle, (N,N-diméthylamino)-carbonylméthyle, propylaminocarbonylméthyle, cyclopropylaminocarbonylméthyle, propénylaminocarbonylméthyle, halogénoéthylaminocarbonylcyclopropyle, 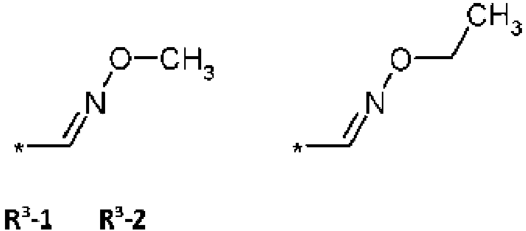 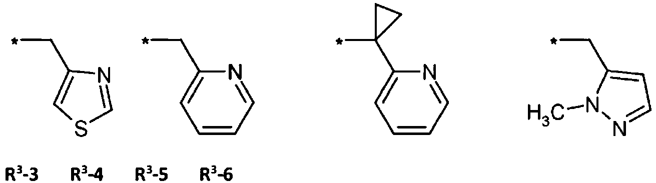 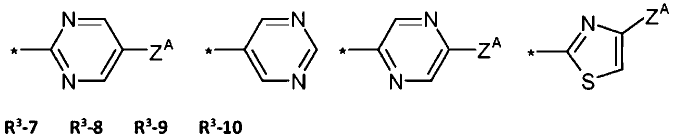 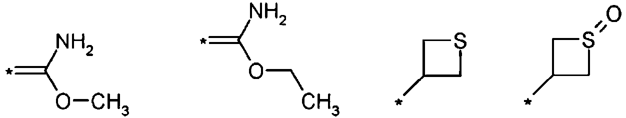 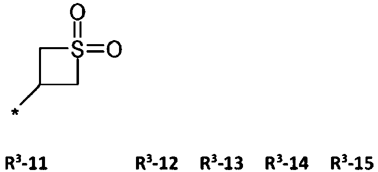
ZA = hydrogène, halogène, cyano, halogénométhyle (CF3) ;
R4 = hydrogène, éthyle, méthoxyméthyle, halogénométhoxyméthyle, éthoxyméthyle, halogénoéthoxyméthyle, propoxyméthyle, méthylcarbonyle, éthylcarbonyle, propylcarbonyle, cyclopropylcarbonyle, méthoxycarbonyle, méthoxyméthylcarbonyle, aminocarbonyle, éthylaminocarbonylméthyle, éthylaminocarbonyléthyle, diméthoxyéthyle, propynylaminocarbonylméthyle, halogénoéthylaminocarbonylméthyle, cyanométhylaminocarbonylméthyle, ou halogénoéthylaminocarbonyléthyle ;
ou
R3 et R4 formant ensemble un substituant choisi dans le groupe constitué par : 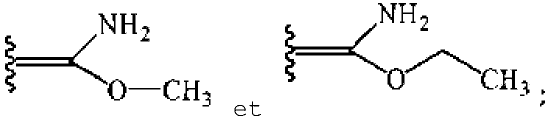 et
(c) un support aqueux ;
les microsphères (a) et les particules de composé (b) étant en suspension dans le support aqueux (c).

2. Composition vétérinaire injectable selon la revendication 1, la lactone macrocyclique étant la moxidectine.

3. Composition vétérinaire injectable selon l'une quelconque des revendications 1 à 2, le D50 de la distribution de tailles de particules pondérée en volume des microsphères (a) étant d'environ 8 µm à environ 250 µm, plus préférablement d'environ 20 µm à environ 200 µm, encore plus préférablement d'environ 40 µm à environ 80 µm, en particulier d'environ 50 µm à environ 70 µm.

4. Composition vétérinaire injectable selon l'une quelconque des revendications 1 à 3, le composé d'isoxazoline selon la formule (I) étant choisi dans le groupe constitué par fluralaner, afoxolaner, sarolaner, et lotilaner.

5. Composition vétérinaire injectable selon l'une quelconque des revendications 1 à 4, le support aqueux comprenant un agent de mise en suspension et l'agent de mise en suspension étant choisi parmi une carboxyméthylcellulose sodique, une polyvinylpyrrolidone, une méthylcellulose et des mélanges correspondants.

6. Composition vétérinaire injectable selon l'une quelconque des revendications 1 à 5, le support aqueux comprenant un agent mouillant et l'agent mouillant étant un poloxamère.

7. Procédé de préparation de la composition vétérinaire injectable selon l'une quelconque des revendications 1 à 6 comprenant les étapes de :
i) préparation de microsphères (a) d'une lactone macrocyclique par évaporation de solvant, atomisation à disque rotatif ou séchage par pulvérisation, et éventuellement tamisage du produit résultant,
ii) préparation de particules (b) du composé d'isoxazoline de formule (I),
iii) préparation du support aqueux par dissolution d'un ou plusieurs agents de mise en suspension et/ou d'un ou plusieurs agents mouillants dans de l'eau, et
iv) mélange des particules obtenues de l'étape i) et des microsphères obtenues de l'étape ii) avec le support aqueux obtenu dans l'étape iii).

8. Procédé selon la revendication 7, la lactone macrocyclique étant la moxidectine et le composé d'isoxazoline selon la formule (I) étant choisi dans le groupe constitué par fluralaner, afoxolaner, sarolaner, et lotilaner.

9. Procédé selon la revendication 7 ou 8, l'agent mouillant étant un poloxamère et l'agent de mise en suspension étant choisi parmi une carboxyméthylcellulose sodique, une polyvinylpyrrolidone, une méthylcellulose et des mélanges correspondants.

10. Kit, le kit comprenant :
(A) un premier récipient comprenant un mélange de microsphères et de particules d'un composé d'isoxazoline de formule (I) tel que défini dans l'une quelconque des revendications 1 à 6,
(B) un deuxième récipient doté d'un support aqueux tel que défini dans l'une quelconque des revendications 1 à 6 ; et
(C) des instructions pour la reconstitution des microsphères et des particules dans le support aqueux avant une injection à un animal.

11. Composition vétérinaire injectable selon les revendications 1 à 6 pour une utilisation dans le traitement et/ou la prévention d'une infestation parasitaire chez un animal.

12. Composition vétérinaire injectable pour une utilisation selon la revendication 11, la composition vétérinaire injectable étant administrée par injection sous-cutanée ou intramusculaire à l'animal.

13. Composition vétérinaire injectable pour une utilisation selon la revendication 11 ou 12, le régime posologique de la composition vétérinaire injectable étant une fois tous les six mois ou une fois tous les douze mois.

14. Composition vétérinaire injectable pour une utilisation selon l'une quelconque des revendications 11 à 13, l'animal étant un animal domestique.

15. Composition vétérinaire injectable pour une utilisation selon la revendication 14, l'animal étant un chien.
